# EUROPEAN PATENT APPLICATION

(11) **EP 0 999 281 A1**
(43) Date of publication of application: **10.05.2000**
(21) Application number: 99124656.2
(22) Date of filing: 24.02.1989
(51) Int. Cl.: C12N 15/86, C12N 5/10, A01K 67/027

(54) **Non-nuclear chromosomal transformation**

(30) Priority: 26.02.1988 US 160771; 26.02.1988 US 160766; 17.02.1989 US 310881
(62) Divisional of application: 89903418.5
(71) Applicant: Biosource Technologies, Inc., Vacaville, CA 95688 (US)
(72) Inventor: Grill, Laurence K., Los Altos, CA 94022 (US); Erwin, Robert L., San Francisco, CA 94107 (US); Berliner, David L., Atherton, CA 94026 (US); Hubbard, Ernest T., Jr., Napa, CA 94558 (US); Turpen, Thomas H., Vacaville, CA 95687 (US)
(74) Representative: Schüssler, Andrea, Dr.

(57) **Abstract**

The present invention relates to viral vectors which are biologically contained, self-replicating and capable of the non-nuclear chromosomal transformation of a host. The viral vectors may contain a heterologous coding sequence adjacent a viral promoter. The invention further relates to viruses containing the viral vectors which a transmissible, i.e. infective. The invention also relates to production cells which are capable of producing the viruses or parts thereof. Host cells are infected by the viruses of the invention. A process for the production of a desired product by growing the infected hosts is also within the scope of the present invention.

## Description

### BACKGROUND OF THE INVENTION

The present invention relates to viral vectors which are non-infective (also referred to herein as biologically contained) but which are self-replicating and capable of the non-nuclear chromosomal transformation of a host. The viral vectors may contain a heterologous coding sequence adjacent a viral promoter. The invention further relates to viruses containing the viral vectors which are transmissible. A host is infected by the viruses of the invention. Production cells are disclosed which are capable of producing the viruses or parts thereof.

Viruses are a unique class of infectious agents whose distinctive features are their simple organization and their mechanism of replication. In fact, a complete viral particle, or virion, may be regarded mainly as a block of genetic material (either DNA or RNA) capable of autonomous replication, surrounded by a protein coat and sometimes by an additional membranous envelope such as in the case of alpha viruses. The coat protects the virus from the environment and serves as a vehicle for transmission from one host cell to another.

Unlike cells, viruses do not grow in size and then divide, because they contain within their coats few or none of the biosynthetic enzymes and other machinery required for their replication. Rather, viruses multiply in cells by synthesis of their separate components, followed by assembly. Thus the viral nucleic acid, after shedding its coat, comes into contact with the appropriate cell machinery where it specifies the synthesis of proteins required for viral reproduction. The viral nucleic acid is then itself replicated through the use of bath viral and cellular enzymes. The components of the viral coat are formed and the nucleic acid and coat components are finally assembled. With some viruses, replication is initiated by enzymes present in virions.

Viruses are subdivided into three main classes --animal viruses, plant viruses and bacterial viruses. Within each class, each virus is able to infect only certain species of cells. With animal and bacterial viruses, the host range is determined by the specificity of attachment to the cells which depends on properties of both the virion's coat and specific receptors on the cell surface. These limitations disappear when transfection occurs, i.e., when infection is carried out by the naked viral nucleic acid, whose entry does not depend on virus-specific receptors.

A given virus may contain either DNA or RNA, which may be either single- or double-stranded. The portion of nucleic acid in a virion varies from about 1% to about 50%. The amount of genetic information per virion varies from about 3 to 300 kb per strand. The diversity of virus-specific proteins varies accordingly. Examples of double-stranded DNA containing viruses include, but are not limited to, Hepatitis B virus, papovaviruses such as polyoma and papilloma, adenovirus, poxviruses such as vaccinia, caulimoviruses such as Cauliflower mosaic virus (CaMV), Pseudomonas phage PMS2, Herpesvirus, Bacillus subtilin phage SP8, and the T bacteriophages. Representative viruses which are single-stranded DNA are the parvoviruses and the bacteriophages φX174, f1 and M13. Reoviruses, cytoplasmic polyhedrosis virus of silkworm, rice dwarf virus and wound tumor virus ate examples of double-stranded RNA viruses. Single-stranded RNA viruses include tobacco mosaic virus (TMV), turnip yellow mosaic virus (TYMV), picornaviruses, myxoviruses, paramyxoviruses and rhabdo-viruses. The RNA in single-stranded RNA viruses may be either a plus (+) or a minus (-) strand. For general information concerning viruses see Grierson, D. et al., Plant Molecular Biology, Blackie, London, pp. 126-146 (1984); Dulbecco, R. et al., Virology, Harper & Row, Philadelphia (1980); White, A. et al., Principles of Biochemistry, 6th Ed., McGraw-Hill, New York, pp. 882-900 (1978).

One means for classifying plant viruses is based on the genome organization. Although many plant viruses have RNA genomes, the organization of genetic information differs between groups. The genome of most monopartite plant RNA viruses is a single-stranded molecule of (+)- sense. There are at least 11 major groups of viruses with this type of genome. An example of this type of virus is TMV. At least six major groups of plant RNA viruses have a bipartite genome. In these, the genome usually consists of two distinct (+)-sense single-stranded RNA molecules that are encapsidated in separate particles. Both RNAs are required for infectivity. Cowpea mosaic virus (CPMW) is an example of a bipartite plant virus. The third major type, containing at least six major types of plant viruses, has three (+)-sense single-stranded RNA molecules, i.e., is tripartite. Each strand is separately encapsidated and all three are required for infectivity. An example of a tripartite plant virus is alfalfa mosaic virus (AMV). Many plant viruses also have smaller sub-genomic mRNAs that are synthesized to amplify a specific gene product. One group of plant viruses which have a single-stranded DNA genome are the geminiviruses, such as cassava latent virus and maize streak virus.

Techniques have been developed which are utilized to transform many species of organisms. Hosts which are capable of being transformed by these techniques include bacteria, yeast, fungus, animal cells and plant cells or tissue. Transformation is accomplished by using a vector which is self-replicating and which is compatible with the desired host. The vectors are generally based on either a plasmid or a virus. Foreign DNA is inserted into the vector, which is then used to transform the appropriate host. The transformed host is then identified by selection or screening. For further information concerning the transformation of these hosts, see Maniatis, T. et al., Molecular Cloning, Cold Spring Harbor Laboratory, Cold Spring Harbor (1982); DNA Cloning, Ed. Glover, D.M., IRL Press, oxford (1985); Grierson, D. et al., supra; and Methods in Enzymology, volumes 68, 100, 101, 118 and 152-155 (1979, 1983, 1983, 1986 and 1987).

Viruses that have been shown to be useful for the transformation of appropriate hosts include bacteriophages, animal viruses such as adenovirus type 2 and vaccinia virus and plant viruses such as CaMV and brome mosaic virus (BMV). An example of the use of a bacteriophage vector is shown in U.S. Patent 4,508,826. U.S. Patent 4,593,002 shows the use of adenovirus type 2 as well as a bacteriophage for the transformation of the appropriate host. The use of a vaccinia virus is shown in U.S. Patent 4,603,112. Transformation of plants using plant viruses is described in EP A 67,553 (TMV), EP A 194,809 (BMV) and Brisson, N. et al., Methods in Enzymology 118, 659 (1986) (CaMV).

When the virus is a DNA virus, the constructions can be made to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

Tyrosinase is a copper-containing monooxygenase catalyzing the ortho hydroxylation of monophenols, including tyrosine and the oxidation of o-diphenols to o-quinones. Mason, H.S., Ann. Rev. Biochem. 34, 595 (1965). Such reactions include the conversion of tyrosine to dihydroxyphenylalanine (dopa) and the conversion of dopa to dopaquinone. These reactions may be followed by the spontaneous formation of melanin. Tyrosinase occurs widely in nature and is responsible for the formation of melanin pigments. Lerch, K., Met.Ions Biol. Syst. 13, 143 (1981).

Tyrosinase is a phenoloxidase found specifically from mammalian sources. Other phenoloxidases or polyphenoloxidases may be found from plant or fungal sources. Phenoloxidases catalyze the oxidation of phenolic compounds (e.g., 3, 4 - dihydroxyphenylalanine (dopa)) to produce melanin (Wheeler, M.H. et al., Can. J. Microbiol. 24, 289 (1978)). A well-defined phenoloxidase is that found in the fungus Cryptococcus neoformans as described by Polachec, I. et al., J. Bacterio. 150, 1212 (1982). Another well-defined phenoloxidase is Drosophilia phenoloxidase described by Rizki, T.M. et al., Mol. Gen. Genet. 201, 7 (1985).

cyclodextrin glucanotransferase is an enzyme catalyzing the conversion of starch to cyclodextrin. Cyclodextrin has several uses including use as a sweetening substance, use as a substitute for gum arabic and other uses in medicines, foods and cosmetics.

### SUMMARY OF THE INVENTION

The present invention relates to viral vectors which are biologically contained, self-replicating and capable of the non-nuclear chromosomal transformation of a host. The viral vectors may contain a heterologous coding sequence adjacent a viral promoter. The invention further relates to viruses containing the viral vectors which are transmissible, i.e. infective.

The invention also relates to production cells which are capable of producing the viruses or parts thereof. Host cells are infected by the viruses of the invention. A process for the production of a desired product by growing the infected hosts is also within the scope of the present invention.

### DETAILED DESCRIPTION OF THE INVENTION

The present invention includes (a) viral vectors which are non-infective but which are self-replicating and capable of the non-nuclear chromosomal transformation of a host, (b) viral vectors which contain a heterologous coding sequence adjacent a viral promoter, (c) viruses containing the viral vectors which are infective, (d) production cells which are capable of producing the viruses or parts thereof, (e) a host infected by the viruses of the invention, and (f) a process for the production of a desired product by growing the infected hosts.

In order to provide a clear and consistent understanding of the specification and the claims, including the scope given to such terms, the following definitions are provided:
Adjacent: A position in a nucleotide sequence immediately 5' or 3' to a defined sequence.
Animal Tissue: Any tissue of an animal in the organism or in culture. This term is intended to include a whole animal, animal cell, animal organ, protoplast, cell culture or any group of animal cells organized into a structural and functional unit.
Anti-sense Mechanism: A type of gene regulation based on controlling the rate of translation of mRNA to protein due to the presence in a cell of an RNA molecule complementary to at least a portion of the mRNA being translated.
Biologically Contained: The viral nucleic acid is not capable of naturally infecting a host since it is not capable of expressing a biologically functional coat protein.
Biolocically Functional: The capability of performing an expected biological function in a cell or organism. For example, the biological function of a viral coat protein is the encapsidation of the viral nucleic acid. A non-biologically functional coat protein is not capable of encapsidating viral nucleic acid. A nucleotide sequence which lacks a biologically functional protein coding sequence will produce either no protein or will produce a protein which will not perform an expected function. If the entire coding sequence for the protein is removed, then no protein will be produced. If a significant portion of the coding sequence for the protein is removed, then any protein that is produced will not function as the entire protein would function. If the coding sequence for the protein is mutated such as by a point mutation, the protein might not function as a normal protein would function. For example, a nucleotide sequence which lacks a biologically functional coat protein coding sequence is a nucleotide sequence which does not code for a coat protein capable of encapsidating viral nucleic acid. This term is intended to include a complete deletion of the coat protein sequence.
Cell Culture: A proliferating mass of cells which may be in an undifferentiated or differentiated state.
Chimeric Sequence or Gene: A nucleotide sequence derived from at least two heterologous parts. The sequence may comprise DNA or RNA.
Coding Sequence: A deoxyribonucleotide sequence which when transcribed and translated results in the formation of a cellular polypeptide, or a ribonucleotide sequence which when translated results in the formation of a cellular polypeptide.
Compatible: The capability of operating with other components of a system. A vector which is compatible with a host is one which is capable of replicating in that host. A coat protein which is compatible with a viral nucleotide sequence is one which is capable of encapsidating the viral sequence.
Gene: A discrete chromosomal region which is rasponsible for a discrete cellular product.
Host: A cell, tissue or organism capable of replicating a viral vector and which is capable of being infected by a virus containing the viral vector. This term is intended to include prokaryotic and eukaryotic cells, organs, tissues or organisms, such as bacteria, yeast, fungus, animal cells and plant tissue.
Infection: The ability of a virus to transfer its nucleic acid to a host wherein the viral nucleic acid is replicated, viral proteins are synthesized and new viral particles assembled. The terms transmissible and infective are used interchangeably herein. The term non-infective as used herein means non-infective by natural, biological means.
Phenotypic Trait: An observable property resulting from the expression of a gene.
Plant Cell: The structural and physiological unit of plants, consisting of a protoplast and the cell wall.
Plant Organ: A distinct and visibly differentiated part of a plant such as root, stem, leaf or embryo.
Plant Tissue: Any tissue of a plant in plant or in culture. This term is intended to include a whole plant, plant cell, plant organ, protoplast, cell culture or any group of plant cells organized into a structural and functional unit.
Production Cell: A cell, tissue or organism capable of replicating a vector or a viral vector, but which is not necessarily a host to the virus. This term is intended to include prokaryotic and eukaryotic cells, organs, tissues or organisms, such as bacteria, yeast, fungus, animal cells and plant tissue.
Promoter: The 5'-flanking, non-coding sequence adjacent a coding sequence which is involved in the initiation of transcription of the coding sequence.
Protoplast: An isolated plant cell without cell walls, having the potency for regeneration into cell culture or a whole plant.
Substantial Sequence Homology: Denotes nucleotide sequences that are substantially functionally equivalent to one another. Nucleotide differences between such sequences having substantial sequence homology will be de minimis in affecting the function of the gene products or an RNA coded for by such sequence.
Transcription: The production of an RNA molecule by RNA polymerase as a complementary copy of a DNA sequence.
Vector: A self-replicating DNA molecule which transfers a DNA segment between cells.
Viral Vector: A vector comprising a nucleic acid sequence of a virus which has been modified so that a non-biologically functional coat protein is produced. This may be accomplished by removing at least a part of the coding sequence or by mutating the coding sequence. If the virus codes for one more virus transmissibility factors, then the nucleic acid sequence of the virus is also modified to make these non-biologically functional.
Virus: An infectious agent which is composed of a nucleic acid encapsidated in a protein. A virus may be a mono-, di-, tri- or multi-partite virus as described above.

The present invention provides for the infection of a host, such as a prokaryotic or eukaryotic organism, cell or tissue, by a virus which has been modified so that the virus is transmissible but the viral nucleic acid is not infective. Naturally occurring mutant viruses may also have these same properties of being transmissible, but having viral nucleic acid which is not infective. The non-infectivity of the viral nucleic acid is accomplished by modifying the nucleic acid so that biologically non-functional viral coat protein (capsid protein) and any other viral transmissibility factors are produced as described herein.

The present invention has a number of advantages, one of which is that the transformation and regeneration of target organisms is not necessary. Another advantage is that it is not necessary to develop vectors which integrate a desired coding sequence in the genome of the target organism. Existing organisms can be altered with a new coding sequence without the need of going through a germ cell. The present invention also gives the option of applying the coding sequence to the desired organism, tissue, organ or cell.

The chimeric genes and vectors of the present invention are constructed using techniques well known in the art. Suitable techniques have been described in Maniatis, T. et al., Molecular Cloning, Cold Spring Harbor Laboratory, New York (1982); Methods in Enzymology, Vols. 68, 100, 101, 118 and 152-155, Academic Press, New York (1979, 1983, 1983, 1986 and 1987); and DNA Cloning, Vols. I, II, III, Glover, D.M., Ed., IRL Press, oxford (1985 and 1987). Medium compositions have been described in Miller, J.H., Experiments in Molecular Genetics, Cold Spring Harbor Laboratory, New York (1972), as well as the references previously identified. DNA manipulations and enzyme treatments are carried out in accordance with the manufacturers' recommended procedures.

An important feature of the present invention is the preparation of nucleotide sequences which are capable of replication in a compatible host but which in themselves are incapable of infecting the host. The nucleotide sequence has substantial sequence homology to a viral nucleotide sequence. The viral nucleotide sequence may be a prokaryotic or eukaryotic viral nucleotide sequence. Suitable viral nucleotide sequences include those of viruses which infect bacteria, yeast, fungus, animals and plants. A partial listing of suitable viruses has been described above. The nucleotide sequence may be an RNA, DNA, cDNA or chemically synthesized RNA or DNA.

The first step in achieving any of the features of the invention is to modify the nucleotide sequences coding for the capsid protein and any transmissibility factors within the viral nucleotide sequence by known conventional techniques such that non-biologically functional proteins are produced by the modified virus. Therefore, any virus for which the capsid protein nucleotide sequence and any transmissibility factor nucleotide sequences have been identified may be suitable for use in the present invention. Other viruses may be used after the nucleic acid has been sequenced.

Some of the viruses which meet this requirement, and therefore are suitable, include the alphaviruses such as Eastern Equine Encephalomyelitis virus (EEEV), Western Equine Encephalomyelitis virus (WEEV). Venezuelan Encephalomyelitis Virus (VEV), Sindbis virus, Semliki Forest virus (SFV) and Ross River virus (RRV), the rhinoviruses such as human rhinovirus 2 (HRV2) and human rhinovirus type 89 (HRV89), the polioviruses such as poliovirus 2 (PV2) and poliovirus 3 (PV3), simian virus 40 (SV40), viruses from the tobacco mosaic virus group such as Tobacco Mosaic virus (TMV), Cowpea Mosaic virus (CMV), Alfalfa Mosaic virus (AmV), Cucumber Green Mottle Mosaic virus watermelon strain (CGMMV-W) and Oat Mosaic virus (OMV) and viruses from the brome mosaic virus group such as Brome Mosaic virus (BMV), broad bean mottle virus and cowpea chlorotic mottle virus. Additional suitable viruses include Rice Necrosis virus (RNV), adenovirus type 2 and geminiviruses such as tomato golden mosaic virus (TGMV), cassava latent virus and maize streak virus. Each of these groups of suitable viruses is characterized below.

### ALPHAVIRUSES

The alphaviruses are a genus of viruses of the family Togaviridae. Almost all of the members of this genus are transmitted by mosquitoes, and may cause diseases in man or animals. Some of the alphaviruses are grouped into three serologically defined complexes. The complex-specific antigen is associated with the E1 protein of the virus, and the species-specific antigen is associated with the E2 protein of the virus.

The Semliki Forest virus complex includes Bebaru virus, Chikungunya Fever virus, Getah virus, Mayaro Fever virus, O'nyongnyong Fever virus, Ross River virus, Sagiyama virus, Semliki Forest virus and Una virus. The Venezuelan Equine Encephalomyelitis virus complex includes Cabassou virus, Everglades virus, Mucambo virus, Pixuna virus and Venezuelan Equine Encephalomyelitis virus. The Western Equine Encephalomyelitis virus complex includes Aura virus, Fort Morgan virus, Highlands J virus, Kyzylagach virus, Sindbis virus, Western Equine Encephalomyelitis virus and Whataroa virus.

The alphaviruses contain an icoschedral nucleocapsid consisting of 180 copies of a single species of capsid protein complexed with a plus-stranded 425 to 498 mRNA of up to about 11,703 nucleotides. The alphaviruses mature when preassembled nucleocapsid is surrounded by a lipid envelope containing two virus encoded integral membrane glycoproteins, called E1 and E2. The envelope is acquired when the capsid, assembled in the cytoplasm, buds through the plasma membrane. The envelope consists of a lipid bilayer derived from the host cell.

The 425 to 498 mRNA encodes a glyprotein which is contranslationally cleaved into nonstructural proteins and structural proteins. The 3' one-third of the RNA genome consists of a 26S mRNA which encodes for the capsid protein and the E3, E2, K6 and E1 glycoproteins. The capsid protein is cotranslationally cleaved from the E3 protein. It is hypothesized that the amino acid triad of His, Asp and Ser at the COOH terminus of the capsid protein comprises a serine protease responsible for cleavage. Hahn, C.S. et al., Proc. Natl. Acad. Sci. U.S.A. 82, 4648 (1985). Cotranslational cleavage also occurs between E2 and K6 proteins. Thus two proteins PE2 which consists of E3 and E2 prior to cleavage and an E1 protein comprising K6 and E1 are formed. These proteins are cotranslationally inserted into the endoplasmic reticulum of the host cell, glycosylated and transported via the Golgi apparatus to the plasma membrane where they can be used for budding. At the point of virion maturation the E3 and E2 proteins are separated. The E1 and E2 proteins are incorporated into the lipid envelope.

It has been suggested that the basic amino-terminal half of the capsid protein stabilizes the interaction of capsid with genomic RNA, Garoff, H. et al., *supra*; or interacts with genomic RNA to initiate encapsidation, Strauss E.G. et al., in the Togaviruses and Flaviviruses, Ed. S. Schlesinger & M. Schlesinger, Plenum Press, New York, p. 35-90, (1980). These suggestions imply that the origin of assembly is located either on the unencapsidated genomic RNA or at the amino-terminus of the capsid protein. It has been suggested that E3 and K6 function as signal sequences for the insertion of PE2 and E1, respectively, into he endoplasmic reticulum. Garoff, H. et al., *supra*; Delgarno, L. et al., Virology 120, 170 (1983).

Work with temperature sensitive mutants of alphaviruses has shown that failure of cleavage of the structural proteins results in failure to form mature virions. Lindquist, B.H. et al., Virology 151, 10 (1986) characterized a temperature sensitive mutant of Sindbis virus, tₛ20. Temperature sensitivity results from an A - U change at nucleotide 9502. The tₛ lesion present cleavage of PE2 to E2 and E3 and the final maturation of progeny virions at the nonpermissive temperature. Hahn, C.S. et al., *supra*, reported three temperature sensitive mutations in the capsid protein which prevents cleavage of the precursor polyprotein at the nonpermissive temperature. The failure of cleavage resulted in no capsid formation and very little envelope protein.

Defective interfering RNA's (DI particles) of Sindbis virus are helper-dependent deletion mutants which interfere specifically with the replication of the homologous standard virus. Perrault, J., Microbiol. Immunol. 93, 151 (1981). DI particles have been found to be functional vectors for introducing at least one foreign gene into cells. Levis, R., Proc. Natl. Acad. Sci. U.S.A. 84, 4811 (1987).

It has been found that it is possible to replace at least 1689 internal nucleotides of a DI genome with a foreign sequence and obtain RNA that will replicate and be encapsidated. Deletions of the DI genome do not destroy biological activity. The disadvantages of the system are that DI particles undergo apparently random rearrangements of the internal RNA sequence and size alterations. Monroe, S.S. et al., J. Virology 49, 865 (1984). Expression of a gene inserted into the internal sequence is not as high as expected. Levis, R. et al., supra, found that replication of the inserted gene was excellent but translation was low. This could be the result of competition with whole virus particles for translation sites and/or also from disruption of the gene due to rearrangement through several passages.

Two species of mRNA are present in alphavirus-infected cells: A 42S mRNA region, which is packaged into nature virions and functions as the message for the nonstructural proteins, and a 26S mRNA, which encodes the structural polypeptides. The 26S mRNA is homologous to the 3' third of the 42S mRNA. It is translated into a 130K polyprotein that is cotranslationally cleaved and processed into the capsid protein and two glycosylated membrane proteins, E1 and E2.

The 26S mRNA of Eastern Equine Encephalomyelitis (EEE) strain 82V-2137 was cloned and analyzed by Chang et al. J. Gen. Virol. 68, 2129 (1987). The 26S mRNA region encodes the capsid proteins, E3, E2, 6K and E1. The amino terminal end of the capsid protein is thought to either stabilize the interaction of capsid with mRNA or to interact with genomic RNA to initiate encapsidation.

Uncleaved E3 and E2 proteins called PE2 is inserted into the host endoplasmic reticulum during protein synthesis. The PE2 is thought to have a region common to at least five alphaviruses which interacts with the viral nucleocapsid during morphogenesis.

The 6K protein is thought to function as a signal sequence involved in translocation of the E1 protein through the membrane. The E1 protein is thought to mediate virus fusion and anchoring of the E1 protein to the virus envelope.

### RHINOVIRUSES

The rhinoviruses are a genus of viruses of the family Picornaviridae. The rhinoviruses are acid-labile, and are therefore rapidly inactivated at pHs less than about 6. The rhinoviruses commonly infect the upper respiratory tract of mammals.

Human rhinoviruses are the major causal agents of the common cold, and many serotypes are known. Rhinoviruses may be propagated in various human cell cultures, and have an optimum growth temperature of about 33°C. Most strains of rhinoviruses are stable at or below room temperature and can withstand freezing. Rhinoviruses can be inactivated by citric acid, tincture of iodine or phenol/alcohol mixtures.

The complete nucleotide sequence of human rhinovirus 2 (HRV2) has been sequenced. The genome consists of 7102 nucleotides with a long open reading frame of 6450 nucleotides which is initiated 611 nucleotides from the 5' end and stops 42 nucleotides from the poly(A) tract. Three capsid proteins and their cleavage cites have been identified.

Rhinovirus RNA is single-stranded and positive sense. The RNA is not capped, but is joined at the 5' end to a small virus encoded protein , virion-protein genome-linked (VPg). Translation is presumed to result in a single polyprotein which is broken by proteolytic cleavage to yield individual virus proteins.

An icosahedral viral capsid contains 60 copies each of 4 virus proteins VP1, VP2, VP3 and VP4 and surrounds the RNA genome. Medappa, K. C. et al. Virology 44, 259 (1971).

Analysis of the 610 nucleotides preceding the long open reading frame shows several short open reading frames. However, no function can be assigned to the translated proteins since only two sequences show homology throughout HRV2, HRV14 and the 3 serotypes of poliovirus. These two sequences may be critical in the life cycle of the virus. They are a stretch of 16 bases beginning at 436 in HRV2 and a stretch of 23 bases beginning at 531 in HRV2. Cutting or removing these sequences from the remainder of the sequence for non-structural proteins could have an unpredictable effect upon efforts to assemble a mature virion.

The capsid proteins of HRV2: VP4, VP2, VP3 and VP1 begin at nucleotide 611, 818, 1601 and 2311, respectively. The cleavage point between VP1 and P2A is thought to be around nucleotide 3255. Skein, T. et al., Nucleic Acids Research 13, 2111 (1985).

Human rhinovirus type 89 (HRV89) is very similar to HRV2. It contains a genome of 7152 nucleotides with a single large open reading frame of 2164 codons. Translation begins at nucleotide 619 and ends 42 nucleotides before the poly(A) tract. The Capsid structural proteins, VP4, VP2, VP3 and VP1 are the first to be translated. Translation of VP4 begins at 619. Cleavage cites occur at:
- VP4/VP2: 825 determined
- VP2/VP3: 1627 determined
- VP3/VP1: 2340 determined
- VP1/P2-A: 3235 presumptive
Duechler, M. et al., Proc. Natl. Acad.Sci. USA 84, 2605 (1987).

### POLIOVIRUSES

Polioviruses are the causal agents of poliomyelitis in man, and are one of three groups of enteroviruses. Enteroviruses are a genus of the family Picornaviridae (also the family of rhinoviruses). Most enteroviruses replicate primarily in the mammalian gastrointestinal tract; although other tissues may subsequently become infected. Many enteroviruses can be propagated in primary cultures of human or monkey kidney cells and in some cell lines (e.g. HeLa, Vero, WI-38). Inactivation of the enteroviruses may be accomplished with heat (about 50°C), formaldehyde (3%), hydrochloric acid (0.1N) or chlorine (ca. 0.3-0.5 ppm free residual Cl₂). The complete nucleotide sequence of poliovirus PV2 (Sab) and PV3 (Sab) have been determined. They are 7439 and 7434 nucleotide in length, respectively. There is a single long open reading frame which begins more than 700 nucleotides from the 5' end. poliovirus translation produces a single polyprotein which is cleaved by proteolytic processing. Kitamura, N. et al. Nature, 291, 547 (1981).

It is speculated that these homologous sequences in the untranslated regions play an essential role in viral replication such as:
1. viral-specific RNA synthesis;
2. viral-specific protein synthesis; and
3. packaging
Toyoda, H. et al., J. Mol. Biol., 174, 561 (1984).

The structures of the serotypes of poliovirus have a high degree of sequence homology. Their coding sequences the same proteins in the same order. Therefore, genes for structural proteins are similarly located. In PV1, PV2 and PV3, the polyprotein begins translation near the 750 nucleotide. The four structural proteins VP4, VP2, VP3 and VP1 begin at about 745, 960, 1790 and 2495, respectively, with VPI ending at about 3410. They are separated in vivo by proteolytic cleavage, rather than by stop/start codons.

### SIMIAN VIRUS 40

Simian virus 40 (SV40) is a virus of the genus Polyomavirus, and was originally isolated from the kidney cells of the rhesus monkey. The virus is commonly found, in its latent form, in such cells. Simian virus 40 is usually non-pathogenic in its natural host.

Simian virus 40 virions are made by the assembly of three structural proteins, VP1, VP2 and VP3. Girard, M. et al., Biochem. Biophys. Res. Commun. 40, 97 (1970); Prives, C. L. et al., Proc. Natl. Acad. Sci. USA 71, 302 (1974); and Rozenblatt, S. et al., Proc. Natl. Acad. Sci. USA 73, 2747 (1976). The three corresponding viral genes are organized in a partially overlapping manner. They constitute the late genes portion of the genome. Tooze, J., Molecular Biology of Tumor Viruses, 2nd Ed. Part 2, p. 799-831 (1980). Cold Spring Harbor Laboratory, Cold Spring Harbor, N.Y. Capsid proteins VP2 and VP3 are encoded by nucleotides 545 to 1601 and 899 to 1601, respectively, and both are read in the same frame. VP3 is therefore a subset of VP2. Capsid protein VP1 is encoded by nucleotides 1488-2574. The end of the VP2-VP3 open reading frame therefore overlaps the VP1 by 113 nucleotides but is read in an alternative frame. Tooze, J., supra. Sychowski, C. et al., J. Virology 61, 3862 (1987)

### TOBACCO MOSAIC VIRUS GROUP

Tobacco Mosaic virus (TMV) is a type member of the Tobamoviruses. The TMV virion is a tubular filament, and comprises coat protein subunits arranged in a single right-handed helix with the single-stranded RNA intercalated between the turns of the helix. TMV infects tobacco as well as other plants. TMV is transmitted mechanically and may remain infective for a year or more in soil or dried leaf tissue.

The TMV virions may be inactivated by subjection to an environment with a pH less than 3 or greater than 8, or by formaldehyde or iodine. Preparations of TMV may be obtained from plant tissues by (NH₄)₂SO₄ precipitation followed by differential centrifugation.

The TMV single-stranded RNA genome is about 6400 nucleotides long and is capped at the 5' end but is not poly-adenylated. The genomic RNA can serve as mRNA for a protein of molecular weight about 130,000 (130K) and another produced by read-through of molecular weight about 180,000 (180K). However, it cannot function as a messenger for the synthesis of coat protein. Other genes are expressed during infection by the formation of monocistronic, 3'-coterminal subgenomic mRNAs, including one (LMC) encoding the 17.5K coat protein and another (I₂) encoding a 30K protein. The 30K protein has been detected in infected protoplasts (Virology 132, 71 (1984)), and it is involved in the cell-to-cell transport of the virus in an infected plant (Deom, C.M. et al., Science 237, 389 (1987)). The functions of the two large proteins are unknown.

Several double-stranded RNA molecules, including double-stranded RNAs corresponding to the genomic, I₂ and LMC RNAs, have been detected in plant tissues infected with TMV. These RNA molecules are presumably intermediates in genome replication and/or mRNA synthesis - processes which appear to occur by different mechanisms.

TMV assembly apparently occurs in the plant cell cytoplasm, although it has been suggested that some TMV assembly may occur in chloroplasts since transcripts of ctDNA have been detected in purified TMV virions. Initiation of TMV assembly occurs by interaction between ring-shaped aggregates ("discs") of coat protein (each disc consisting of two layers of 17 sub-units) and a unique internal nucleation site in the RNA; a hairpin region about 900 nucleotides from the 3' end in the common strain of TMV. Any RNA, including subgenomic RNAs, containing this site may be packaged into virions. The discs apparently assume a helical form on interaction with the RNA, and assembly (elongation) then proceeds in both directions (but much more rapidly in the 3'- to 5' direction from the nucleation site).

Another member of the Tobamoviruses, the Cucumber green mottle mosaic virus watermelon strain (CGMMV-W) is related to the cucumber virus. Noru, Y., et al., Virology 45, 577 (1971). The coat protein of CGMMV-W interacts with the RNA of both TMV and CGMMV to assemble viral particles in vitro. Kurisu et al., Virology 70, 214 (1976).

Several strains of the tobamovirus group are divided into two subgroups on the basis of the location of the assembly of origin. Fukuda, M. et al., Proc. Natl. Acad. Sci. USA 78, 4231 (1981). Subgroup I, which includes the vulgare, OM, and tomato strain, has an origin of assembly at about 800-1000 nucleotides from the 3' end of the RNA genome, and outside of the coat protein cistron. Lebeurier, G. et al., Proc. Natl. Acad. Sci. USA 74, 1913 (1977); and Fukuda, M. et al., Virology 101, 493 (1980). Subgroup II, which includes CGMMV-W and cornpea strain (Cc), has an origin of assembly about 300-500 nucleotides from the 3' end of the RNA genome, and within the coat-protein cistron. Fukuda, M. et. al., supra. The coat protein cistron of CGMMV-W is located at nucleotides 176-661 from the 3'end. The 3' noncoding region is 175 nucleotides long. The origin of assembly is positioned within the coat protein cistron. Meshi, T. et al., Virology 127, 52 (1983).

### BROME MOSAIC VIRUS GROUP

Brome mosaic virus (BMV) is a member of a group of tripartite single-stranded RNA-containing plant viruses commonly referred to as the bromoviruses. Each member of the bromoviruses infects a narrow range of plants. Mechanical transmission of bromoviruses occurs readily, and some members are transmitted by beetles. In addition to BMV, other bromoviruses include broad bean mottle virus and cowpea chlorotic mottle virus.

Typically, a bromovirus virion is icosahedral with a diameter of about 26 mm., and contains a single species of coat protein. The bromovirus genome has three molecules of linear, positive-sense, single-stranded RNA, and the coat protein mRNA is also encapsidated. The RNAs each have a capped 5' end and a tRNA-like structure (which accepts tyrosine) at the 3' end. Virus assembly occurs in the cytoplasm. The complete nucleotide sequence of BMV has been identified and characterized as described by Alquist et al., J. Mol. Biol. 153, 23 (1981).

### RICE NECROSIS VIRUS

Rice Necrosis virus is a member of the Potato Virus Y Group or Potyviruses. The Rice Necrosis virion is a flexuous filament comprising one type of coat protein (molecular weight about 32,000 to about 36,000) and one molecule of linear positive-sense single-standard RNA. The Rice Necrosis virus is transmitted by Polymyxa graminis (a eukaryotic intracellular parasite found in plants, algae and fungi).

### ADENOVIRUSES

Adenovirus type 2 is a member of the adenovirus family or adenovirus. This family of viruses are non-enveloped, icosahedral, linear, double-stranded DNA-containing viruses which infect mammals or birds.

The adenovirus virion consists of an icosahedral capsid enclosing a core in which the DNA genome is closely associated with a basic (arginine-rich) viral polypeptide VII. The capsid is composed of 252 capsomeres: 240 hexons (capsomers each surrounded by 6 other capsomers) and 12 pentons (one at each vertex, each surrounded by 5 'peripentonal' hexons). Each penton consists of a penton base (composed of viral polypeptide III) associated with one (in mammalian adenoviruses) or two (in most avian adenoviruses) glycoprotein fibres (viral polypeptide IV). The fibres can act as haemagglutinins and are the sites of attachment of the virion to a host cell-surface receptor. The hexons each consist of three molecules of viral polypeptide II; they make up the bulk of the icosahedron. Various other minor viral polypeptides occur in the virion.

The adenovirus dsDNA genome is covalently linked at the 5' end of each strand to a hydrophobic 'terminal protein', TP (molecular weight about 55,000); the DNA has an inverted terminal repeat of different length in different adenoviruses. In most adenoviruses examined, the 5'-terminal residue is dCMP.

During its replication cycle, the virion attaches via its fibres to a specific cell-surface receptor, and enters the cell by endocytosis or by direct penetration of the plasma membrane. Most of the capsid proteins are removed in the cytoplasm. The virion core enters the nucleus, where the uncoating is completed to release viral DNA almost free of virion polypeptides. Virus gene expression then begins. The viral dsDNA contains genetic information on both strands. Early genes (regions E1a, E1b, E2a, E3, E4) are expressed before the onset of viral DNA replication. Late genes (regions L1, L2, L3, L4 and L5) are expressed only after the initiation of DNA synthesis. Intermediate genes (regions E2b and IVa₂) are expressed in the presence or absence of DNA synthesis. Region E1a encodes proteins involved in the regulation of expression of other early genes, and is also involved in transformation. The RNA transcripts are capped (with m⁷G⁵ppp⁵N) and polyadenylated in the nucleus before being transferred to the cytoplasm for translation.

Viral DNA replication requires the terminal protein, TP, as well as virus-encoded DNA polymerase and other viral and host proteins. TP is synthesized as an 80K precursor, pTP, which binds covalently to nascent replicating DNA strands. pTP is cleaved to the mature 55K TP late in virion assembly; possibly at this stage, pTP reacts with a dCTP molecule and becomes covalently bound to a dCMP residue, the 3' OH of which is believed to act as a primer for the initiation of DNA synthesis. Late gene expression, resulting in the synthesis of viral structural proteins, is accompanied by the cessation of cellular protein synthesis, and virus assembly may result in the production of up to 10⁵ virions per cell.

### GEMINIVIRUSES

Geminiviruses are a group of small, single-stranded DNA-containing plant viruses with virions of unique morphology. Each virion consists of a pair of isometric particles (incomplete icosahedra), composed of a single type of protein (molecular weight about 2.7-3.4 x 10⁴). Each geminivirus virion contains one molecule of circular, positive-sense, single-stranded DNA. In some geminiviruses (i.e., cassava latent virus and bean golden mosaic virus), the genome appears to be bipartite, containing two single-stranded DNA molecules which are of similar size, but differ as to nucleotide sequence. However, other geminiviruses (i.e., the leafhopper transmitted viruses such as Chloris striate mosaic virus), have only one type of single-stranded DNA. Geminivirus replication occurs in the plant cell nucleus where large aggregates of virus particles accumulate.

The nucleotide sequence of any suitable virus can be derived from a viral nucleic acid by modifying the coat protein coding sequence. The modification may be the removal of a coding sequence for at least a part of the viral coat protein. Alternatively, the nucleotide sequence can be synthesized such that it lacks at least a part of the viral coat protein coding sequence. A sufficient amount of the coding sequence is removed such that any coat protein which may be produced by the virus will be incapable of encapsidating the viral nucleic acid. In addition, the coat protein coding sequence may be modified by mutation such that the coat protein which is produced is incapable of encapsidating the viral nucleic acid. In each instance, as non-biologically functional protein is produced. In some instances, part of the gene may be necessary for good promoter activity. If so, then the entire gene should not be deleted. In order to be easily transmissible to other plants, the nucleotide sequence must be encapsidated in a compatible coat protein as described further below. The viral nucleic acid may further be modified to alter the coding sequence for any viral transmissibility factors. The alteration of the coding sequences for these factors will ensure that the nucleotide sequence cannot be transmitted by other vectors, e.g. by insects.

The nucleotide sequence is prepared by cloning the viral nucleic acid in an appropriate production cell. If the viral nucleic acid is DNA, it can be cloned directly into a suitable vector using conventional techniques. One technique is to attach an origin of replication compatible with the production cell to the viral DNA. If the viral nucleic acid is RNA, a full-length DNA copy of the viral genome is first prepared by well known procedures. For example, the viral RNA is transcribed into DNA using reverse transcriptase to produce subgenomic DNA pieces, and a double-stranded DNA made using DNA polymerases. The DNA is then cloned into appropriate vectors and cloned into a production cell. The DNA pieces are mapped and combined in proper sequence to produce a full-length DNA copy of the viral RNA genome. The coding sequences for the viral coat protein and any viral transmissibility factors are identified and altered. The resulting nucleotide sequence is self-replicating but incapable of infecting host itself. Any manner of separating the coding sequences for the viral coat protein and the coding sequences for the viral transmissibility factors from the remainder of the viral nucleotide sequence or of altering the nucleotide sequences of these proteins to produce non-biologically functional proteins is suitable for the present invention.

In the case of alphaviruses, the E1 and E2 glycoproteins may play a role in transmissibility of the virus (Garaff, H. et al., Nature 288, 236 (1980)). These glycoproteins are incorporated in a liquid envelope which surrounds the coat protein. The nucleotide sequence which codes for the E1 and E2 glycoproteins is adjacent to the coding sequence for the coat protein in alphavirus RNA. Therefore the E1 and E2 glycoprotein coding sequences can be removed with the coat protein coding sequence by known conventional techniques.

A second feature of the present invention is a chimeric nucleotide sequence which comprises a first nucleotide sequence and a second nucleotide sequence. The first sequence is capable of self-replication, is not capable of transmission and has substantial sequence homology to a viral nucleotide sequence, as described above. The second sequence is capable of being transcribed in a host. The second sequence is preferably placed adjacent a viral promoter, although a fusion protein may be produced which also has biological activity. Any viral promoter can be utilized, but it is preferred to use a promoter of the viral coat protein gene, at least a part of the coding sequence of which has been deleted. In those instances where the coat protein coding sequence is altered but not deleted, a viral promoter can be attached to the second sequence by conventional techniques or the second sequence can be inserted into or adjacent the coat protein coding sequence such that a fusion protein is produced. The second sequence which is transcribed may be transcribed as an RNA which is capable of regulating the expression of a phenotypic trait by an anti-sense mechanism. Alternatively, the second sequence in the chimeric nucleotide sequence may be transcribed and translated in the host, e.g., plant tissue, to produce a phenotypic trait. The second nucleotide sequence may also code for the expression of more than one phenotypic trait. The chimeric nucleotide sequence is constructed using conventional techniques such that the second nucleotide sequence is in proper orientation to the viral promoter.

Useful phenotypic traits in plant cells include, but are not limited to, improved tolerance to herbicides, improved tolerance to extremes of heat or cold, drought, salinity or osmotic stress; improved resistance to pests (insects, nematodes or arachnids) or diseases (fungal, bacterial or viral); production of enzymes or secondary metabolites; male or female sterility; dwarfness; early maturity; improved yield, vigor, heterosis, nutritional qualities, flavor or processing properties, and the like. Other examples include the production of important proteins or other products for commercial use, such as lipase, melanin, pigments, antibiotics and the like. Another useful phenotypic trait is the production of degradative or inhibitory enzymes, such as are utilized to prevent or inhibit root development in malting barley. The phenotypic trait may also be a secondary metabolite whose production is desired in a bioreactor.

In the case of animal cells, useful phenotypic traits include, but are not limited to, the ability to grow in culture, the elimination of the characteristic of attaching to a substrate when grown in culture, enhanced immune response, minimization of inappropriate immune responses such as autoimmune reactions, more efficient metabolism, increased fat and lipid metabolism for the production of leaner meats, replacement of deficient enzymes and better utilization of feed. Like plant cells, other examples of useful phenotypic traits for animal cells include the production of important proteins or other products for commercial use, such as lipase, melanin, pigments, antibiotics, tissue plasminogen activator (TPA), human growth hormone and the like, or a secondary metabolite whose production is desired in a bioreactor.

An example of a chimeric nucleotide sequence is one which contains a first nucleotide sequence having substantial sequence homology to TMV and a second nucleotide sequence which is a coding sequence for tyrosinase. In a second example, the virus is oat mosaic virus (OMV) or rice necrosis virus (RNV). OMV and RNV are capable of infecting most monocot species including, but not limited to, barley and corn. In a third example, the second nucleotide sequence is the coding sequence for cyclodextrin glucanotransferase. Potato virus Y (PVY) or potato virus X (PVX) is used in a fourth example. In a fifth example, a chimeric nucleotide sequence contains a first nucleotide sequence having substantial sequence homology to gemini tomato golden mosaic virus (TGMV), and a second nucleotide sequence which codes for human tissue plasminogen activator (t-PA). t-PA is isolated from plasmid pt-PAtrp12, ATCC No. 40404 (U.S. Patent 4,766,075). TGMV is capable of infecting a wide variety of both dicotyledonous and monocotyledonous plants including tobacco, tomato, bean, soya bean, sugar beet, cassava, cotton, maize, oats and wheat. Many other examples are illustrated herein.

The second nucleotide sequence can be inserted into the first nucleotide sequence prepared above such that it is adjacent a viral promoter. Since the location of the promoter of the viral coat protein gene is known in this sequence as a result of the deletion of the gene, the second nucleotide sequence can be placed adjacent this promoter. Alternatively, an appropriate viral promoter can first be attached to the second nucleotide sequence and this construct can then be inserted either into the first nucleotide sequence or adjacent thereto. In addition, the second nucleotide sequence can be inserted into and adjacent an altered coat protein coding sequence.

A double-stranded DNA of the chimeric nucleotide sequence or of a complementary copy of the chimeric nucleotide sequence is cloned into a production cell. If the viral nucleic acid is an RNA molecule, the chimeric nucleotide sequence is first attached to a promoter which is compatible with the production cell. The chimeric nucleotide sequence can then be cloned into any suitable vector which is compatible with the production cell. In this manner, only RNA copies of the chimeric nucleotide sequence are produced in the production cell. For example, if the production cell is E. coli, the lac promoter can be utilized. If the production cell is a plant cell, the CaMV promoter can be used. The production cell will be a eukaryotic cell such as yeast, plant or animal, if viral RNA must be capped for biological activity. The chimeric nucleotide sequence can then be cloned into any suitable vector which is compatible with the production cell. Alternatively, the chimeric nucleotide sequence is inserted in a vector adjacent a promoter which is compatible with the production cell. If the viral nucleic acid is a DNA molecule, it can be cloned directly into a production cell by attaching it to an origin of replication which is compatible with the production cell. In this manner, DNA copies of the chimeric nucleotide sequence are produced in the production cell.

In the case of alphaviruses, where the E1 and E2 glycoproteins and the coat protein are removed, a larger foreign protein coding sequence may be inserted to form the chimeric nucleotide sequence. The E1 and E2 glycoproteins do not have their own promoters, so the coat protein promoter would be used for a foreign coding sequence inserted in place of the adjacent coding sequences for the coat protein, E1 glycoprotein and E2 glycoprotein.

Alternatively, more than one foreign coding sequence may be inserted in place of the adjacent coding sequences for the coat protein, E1 glycoprotein and E1 glycoprotein. However, in this case, each foreign coding sequence would require its own appropriate viral promoter. However, the coat protein promoter could be used for one foreign coding sequence if that promoter had been preserved in the nucleotide sequence.

A promoter is a DNA sequence that directs RHA polymers to bind to DNA and to initiate RNA synthesis. There are strong promoters and weak promoters. Among the strong promoters are lacuv5, trp, tac, trp-lacuv5, λp₁, ompF, and bla. A useful promoter for expressing foreign genes in E. coli is both strong and regulated. The λp₁ promoter of bacteriophage λ is a strong, well-regulated promoter. Hedgpeth, J.M. et al. Mol. Gen. Genet. 163, 197 (1978); Bernard, H. M. et al. Gene 5, 59 (1979); Remaut, E.P. et al., Gene 15, 81 (1981).

A gene encoding a temperature-sensitive λ repressor such as λ*cITs* 857 may be included in the cloning vector. Bernard et al., supra. At low temperature (31°C), the p₁ promoter is maintained in a repressed state by the cI-gene product. Raising the temperature destroys the activity of the repressor. The p₁ promoter then directs the synthesis of large quantities of mRNA. In this way, E. coli production cells may grow to the desired concentration before producing the products encoded within the vectors. Similarly, a temperature-sensitive promoter may be activated at the desired time by adjusting the temperature of the culture.

It may be advantageous to assemble a plasmid that can conditionally attain very high copy numbers. For example, the pAS2 plasmid containing a lac or tac promoter will achieve very high copy numbers at 42°C. The lac repressor, present in the pAS2 plasmid is then inactivated by isopropyl-β-D-thiogalactoside to allow synthesis of mRNA.

A further alternative when creating the chimeric nucleotide sequence, is to prepare mare than one nucleotide sequence (i.e., prepare the nucleotide sequences necessary for a multipartite viral vector construct). In this case, each nucleotide sequence would require its own origin of assembly. Each nucleotide sequence could be chimeric (i.e., each nucleotide sequence has a foreign coding sequence inserted therein), or only one of the nucleotide sequences may be chimeric.

If a multipartite virus were found to have the coding sequence for its coat protein on one strand of nucleic acid, and the coding sequence for a transmissibility factor on a different strand, then two chimeric nucleotide strands would be created in accordance with the invention. One foreign coding sequence would be inserted in place of the coat protein gene (or inserted next to the altered coat protein gene) on one strand of nucleic acid, and another foreign coding sequence would be inserted in place of the transmissibility factor gene (or inserted next to the altered transmissibility factor gene) on the other strand of nucleic acid.

Alternatively, the insertion of a foreign coding sequence into the nucleotide sequence of a monopartite virus may result in the creation of two nucleotide sequences (i.e., the nucleic acid necessary for the creation of a bipartite viral vector). This would be an advantageous situation when it is desirable to keep the replication and translation of the foreign coding sequence separate from the replication and translation of some of the coding sequences of the original nucleotide sequence. Each nucleotide sequence would have to have its own origin of assembly.

A third feature of the present invention is a virus or viral particle. The virus comprises a chimeric nucleotide sequence as described above which has been encapsidated. The resulting product is then capable of infecting an appropriate host, but the chimeric nucleotide sequence is incapable of further infection since it lacks the mechanism to produce additional virus or viral particles. However, the chimeric nucleotide sequence is capable of replicating in the host. The chimeric nucleotide sequence is transcribed and/or translated within the host to produce the desired product.

Most viruses are encapsidated by either an icosahedral capsid, a spherical capsid or a rod-shaped capsid. Plant viruses such as the tobacco mosaic virus are commonly encapsidated by a rod-shaped capsid, while alphaviruses are encapsidated by an icosahedral capsid.

The icosahedral capsids and the spherical capsids are more geometrically constrained than a rod-shaped capsid, and therefore are more limited as to the amount of nucleic acid which may be encapsidated. In contrast, a rod-shaped capsid is expandable so that it can encapsidate any amount of nucleic acid as explained in European Patent Application 0 278 667.

In one embodiment of the present invention, the chimeric nucleotide sequence is encapsidated by a heterologous capsid. Most commonly, this embodiment will make use of a rod-shaped capsid because of its ability to encapsidate a longer chimeric nucleotide sequence than the more geometrically constrained icosahedral capsid or spherical capsid. The use of a rod-shaped capsid permits the incorporation of a larger foreign protein coding sequence to form the chimeric nucleotide sequence. Such a rod-shaped capsid is most advantageous when more than one foreign coding sequence is present in the chimeric nucleotide sequence.

Another feature of the invention is a vector containing the chimeric nucleotide sequence as described above. The chimeric nucleotide sequence is adjacent a nucleotide sequence selected from the group consisting of a production cell promoter or an origin of replication compatible with the production cell. The vector is utilized to transform a production cell which will then produce the chimeric nucleotide sequence in quantity. The production cell may be any cell which is compatible with the vector. The production cell may be prokaryotic or eukaryotic. However, if the viral RNA (chimeric nucleotide sequence) must be capped in order to be active, then the production cell must be capable of capping the viral RNA, such as a eukaryotic production cell. The production cell may contain only the vector which contains the chimeric nucleotide sequence. In this instance, the production cell will only produce the chimeric nucleotide sequence which must then be encapsidated in vitro to produce an infective virus. The encapsidation is accomplished by adding a compatible viral coat protein to the chimeric nucleotide sequence in accordance with conventional techniques. In this scenario, the viral coat protein would be produced in a second production cell. A second vector comprising a coding sequence for a compatible viral coat protein adjacent a promoter compatible with the production cell is utilized to transform the second production cell. The second production cell then produces the viral coat protein in quantity.

As explained above, the viral coat protein produced by the second production cell may be homologous or heterologous to the viral nucleotide sequence. The viral coat protein must be compatible with the chimeric nucleotide sequence and of sufficient size to completely encapsidate the chimeric nucleotide sequence.

Alternatively, the first production cell further contains a second vector which comprises a coding sequence for a compatible viral coat protein adjacent a promoter compatible with the production cell. Again, this coding sequence for the viral coat protein may be homologous or heterologous to the viral nucleotide sequence. In this instance, the production cell then produces the coat protein and the chimeric nucleotide sequence. The chimeric nucleotide sequence is then encapsidated by the viral coat protein to produce a transmissible virus. The second vector is prepared by conventional techniques, by inserting the coding sequence for the viral coat protein which was deleted in the preparation of the chimeric nucleotide sequence, or a homologous or heterologous viral coat protein coding sequence into an appropriate vector under the control of a promoter compatible with the production cell.

There are numerous alternatives whereby different vectors are utilized to transform separate production cells or the same production cell. With most viruses, it is unknown whether transmissibility factors alone are essential for the infectivity of a virion or whether the transmissibility factors simply serve as an adjunct to the capsid, and therefore enhance virion infectivity, but do not prevent infection if not present on the virion. For purposes of this invention (i.e., to insure non-infectiousness of the viral vector), it has been assumed that the transmissibility factors alone could render the viral vector infectious, and therefore the vector comprising the chimeric nucleotide sequence will contain neither a nucleotide sequence for the capsid protein nor a nucleotide sequence for any transmissibility factor.

However, this vector comprising the chimeric nucleotide sequence must contain a promoter and an origin of replication, and preferably contains an origin of assembly. As stated above, this vector is utilized to transform a production cell. The origin of replication must be compatible with the production cell.

The other necessary vector or vectors may be varied primarily dependent on the virus of interest. If the viral nucleotide sequence for the capsid protein is adjacent the nucleotide sequence for the transmissibility factors, and the sequences share the same promoter (as in the case with alphaviruses), a second vector comprises the sequence for the capsid protein, the sequence for the transmissibility factors and a promoter. This second vector may optionally contain the origin of assembly if the origin of assembly has not been incorporated in the first vector. When this vector is utilized to transform a production cell, the origin of assembly must be compatible with that production cell.

Alternatively, the second vector may comprise the nucleotide sequence for the capsid protein and a promoter, and a third vector may comprise the nucleotide sequence for the transmissibility factors and a promoter. Either the second or third vector could further comprise the origin of assembly which would have to be compatible with the production cell to be transformed by the vector.

With any of the alternative embodiments, each vector may be utilized to transform its own production cell, or all of the vectors may be utilized in one production cell. All of the promoters associated with each vector must also be compatible with the production cell in which the vector is utilized.

As previously discussed, a prokaryotic production cell can be transformed to contain a vector containing the coat protein coding sequence or any other coding sequence necessary for infectivity of the virus. Alternatively, the prokaryotic production cell or a eukaryotic production cell is transforms such that the coding sequences for the coat protein and transmissibility factors (if necessary) are stably incorporated into the genome of the cell. Several conventional techniques can be utilized to accomplish this embodiment. For example, appropriate coding sequences can be inserted into plant cells by transformation with Agrobacterium, such as described by Schell, J. et al., Bio/Technology 1, 175 (1983); Fillatti J. et al., Bio/Technology 5, 726 (1987); Everett, N.P. et al., Bio/Technology 5, 1201 (1987); Pua, E-C., Bio/Technology 5, 815 (1987); Hinchee, M.A., et al., Bio/Technology 6, 915 (1988) and Meth. Enzymol, Vol. 118, supra. Agrobacterium have also been utilized for introducing viruses into plants, both dicots and monocots by a process termed Agro-infection. This technique has been described by Grimsley N., et al. Proc. Natl. Acad. Sci. USA 83, 3282 (1986); Elmer, J.s. et al., Plant Mol Biol 10, 225 (1988); Grimsley N. et al., Nature 325, 177 (1987); and Hayes, R.J. et al., Nature 334, 180 (1988).

Alternatively, the appropriate coding sequence can be inserted into eukaryotic cells by direct gene transfer including electroporation calcium chloride or Polyethylene glycol mediated transformation liposome fusion microinjection or microprojectile bombardment. These techniques have been described by Fromm, M.E., Meth. Enzymol 153, 307 (1987); Shillito, R.D. et al., Meth. Enzymol 153, 283 (1987); Dehayes, A., et al., EMBO J 4, 2731 (1985); Negrutin, R. et al., Plant Mol Biol 8, 363 (1987); Reich, T.J. et al., Bio/Technology 4, 1001 (1986); Klein, T.M. et al., Bio/Technology 6, 559 (1988); McCabe, D.E. et al., Bio/Technology 6, 923 (1988).

A production cell which has been transformed will contain the coat protein and/or transmissibility factors coding sequence(s) either in a stable vector or stably incorporated in the genome. The vector containing the chimeric nucleotide sequence is then introduced into the production cell as previously discussed.

Once the chimeric nucleotide sequence is replicated and the capsid protein and transmissibility factors have been produced by the appropriate production cells, the intact virions may be assembled in vitro. The replicated chimeric nucleotide sequence is encapsidated by the capsid protein in accordance with known conventional techniques. The transmissibility factors may be added to the virion assembly separately when a third vector is used to cause the production of transmissibility factors separate from the capsid protein. Alternatively, if a single second vector was used to cause the production of both capsid protein and transmissibility factors (as is usually the case with alphaviruses), then the in vitro virion assembly is complete upon encapsidation of the chimeric nucleotide sequence.

A further feature of the present invention is a host which has been infected by the virus. After introduction into a host, the host contains the chimeric nucleotide sequence which is capable of self-replication but which is not capable of producing additional transmissible viruses or viral particles. The host can be infected with the virus by conventional techniques. Suitable techniques include, but are not limited to, leaf abrasion, abrasion in solution, high velocity water spray and other injury of a host as well as imbibing host seeds with water containing the chimeric nucleotide sequence alone or the encapsidated virus. Although the chimeric nucleotide sequence is not capable of producing infective agents, it is capable of self-replicating and spreading throughout the host, e.g. if the host is a plant or animal.

An alternative method for introducing a chimeric nucleotide sequence into a plant host is a technique known as agroinfection or Agrobacterium-mediated transformation (sometimes called Agro-infection) as described by Grimsley, N. et al., Nature, 325, 177 (1987). This technique makes use of a common feature of Agrobacterium which colonize plants by transferring a portion of their DNA (the T-DNA) into a host cell, where it becomes integrated into nuclear DNA. The T-DNA is defined by border sequences which are 25 base pairs long, and any DNA between these border sequences is transferred to the plant cells as well. The insertion of a chimeric viral nucleotide sequence between the T-DNA border sequences results in transfer of the chimeric sequence to the plant cells, where the chimeric sequence is replicated, and then spreads systemically through the plant. Agro-infection has been accomplished with potato spindle tuber viroid (PSTV) (Gardner, R.C. et al., Plant Mol. Biol. 6, 221 (1986)), cauliflower mosaic virus (CaMV) (Grinsley, N. et al., Proc. Natl. Acad. Sci. U.S.A. 83, 3282 (1986)), maize streak virus (Grimsley, N. et al., Nature 325, 177 (1987) and Lazarowitz, S.G., Nucl. Acids Res. 16, 229 (1988)), digitaria streak virus (Donson, J. et al., Virogology 162, 248 (1988)), wheat dwarf virus (Hayes, R.J. et al., J. Gen. Virol. 69, 891 (1988)) and tomato golden mosaic virus (TGMV) (Elmer, J.S. et al., Plant Mol. Biol. 10, 225 (1988) and Gardiner, W.E. et al., EMBO J. 7, 899 (1988)). Therefore, agro-infection of a susceptible plant could be accomplished with a virion containing a chimeric nucleotide sequence based on the nucleotide sequence of any of the above viruses.

Several viral products are useful to insure the production and spread of viral nucleic acids. One factor is a replicase which is involved in the replication of the viral nucleic acid. A second factor which may be present is termed herein a transport protein. This protein(s) is(are) involved in the movement of the viral nucleic acid from infected cells to adjacent cells and thus, the spread of the viral nucleic acid throughout the production or host cell, tissue or organism. In order to insure suitable replication and spread of the viral vector, an additional embodiment of the present invention includes the stable transformation of the host or production cell, tissue or organism with a coding sequence for viral replicase, a viral transport protein or both. The transformation, including stable integration into the genome of the production cell or host, is accomplished as described above concerning the coat protein gene.

In an additional embodiment of the invention, the host range of a virus is enlarged so that a viral construct, i.e., chimeric nucleic acid, containing different foreign genes may be used in many different hosts. The host range of the virus is enlarged by transforming the host (or production cell since it will also work in this context) to contain parts of viral material useful in enabling infection of the host by the viral construct. For animal or bacterial hosts, the hosts are transformed to contain coding sequence(s) for the host cell receptors recognized by the virus. Alternatively, the virus can be produced to contain coding sequences of the recognition or binding proteins of the host. For plant hosts, the hosts are transformed to contain the viral replicase gene or transport protein gene. Alternatively, the virus can be produced to contain coding sequences for transport proteins capable of functioning in the host. In addition, it is possible to transform protoplasts of the host plant to contain the viral nucleic acid or parts thereof such that all cells of the regenerated plants will be capable of infection. This transformation is carried out by any of the techniques previously described.

A still further feature of the invention is a process for the production of a specified product such as, but not limited to, an anti-sense RNA, a ribozyme, a protein, an enzyme or a complex biomolecule. Such products include those discussed above. The second nucleotide sequence of the chimeric nucleotide sequence comprises the transcribable sequence which leads to the production of the desired product. This process involves the infection of the appropriate host with a virus, such as those described above, the growth of the infected host to produce the desired product and the isolation of the desired product, if necessary. The growth of the infected host is in accordance with conventional techniques, as is the isolation of the resultant product.

In one example, a tyrosinase coding sequence such as isolated from Streptomyces antibioticus is inserted adjacent the promoter of the viral coat protein of a nucleotide sequence derived from TMV, oat mosaic virus (OMV) or rice necrosis virus (RNV) in which the coat protein coding sequence has been removed. A virus is prepared as described above using the resulting chimeric nucleotide sequence. Tobacco or germinating barley is infected with the appropriate virus. The chimeric nucleotide sequence self-replicates in the plant tissue to produce the enzyme tyrosinase. Alternatively, the tyrosinase coding sequence is inserted into other appropriately modified viral nucleic acid. The resultant virus is used to infect the appropriate host and tyrosinase is produced. The activity of this enzyme leads to the production of melanin. See for example. Huber, M. et al., Biochemistry 24, 6038 (1985).

In a second example, a cyclodextrin glucanotransferase coding sequence, such as isolated from Bacillus sp. N. 17-1 (see U.S. Patent 4,135,977) is inserted adjacent the promoter of the viral coat protein of a nucleotide sequence derived from OMV, RNV, PVY or PVX in which the coat protein coding sequence has been removed. A virus is prepared as described above using the resulting chimeric nucleotide sequence. Corn or potato is infected with the appropriate virus. The chimeric nucleotide sequence self-replicates in the plant tissue to produce the enzyme cyclodextrin glucotransferase. The activity of this enzyme leads to the production of cyclodextrin, which is useful as flavorant or for drug delivery.

### EXAMPLES

The following examples further illustrate the present invention. In these examples, enzyme reactions were conducted in accordance with the manufacturer's recommended procedures unless otherwise indicated. Standard techniques such as those described in Maniatis, T. et al., supra; Meth. in Enzymol. Vol. 68, 100, 101, 118, 152-155, supra; and DNA Cloning, Vol. I, II, III, supra, were utilized for vector constructions and transformation unless otherwise specified.

### EXAMPLE 1

### Preparation of Chimeric TMV DNA comprising Tyrosinase Gene in Place of the Coat Protein Gene

The 1.2 kb tyrosinase gene was derived from the pIJ702 streptomyces plasmid (available to the public) using a SacI/PVUII cut. It was inserted into the SacI/EcoRV site of the Stratagene bluescript vector (KS), which is 2.9 kb in size. The resulting plasmid was maintained as pBG110 (4.1 kb). The tyrosinase gene was removed from pBG110 by a SacI/HindIII cut and inserted into pUC19 (2.7 kb) giving pBG115 (3.9 kb). The tyrosinase gene was removed from pBG115 by a EcoRI/HindIII cut and inserted into the Stratagene bluescript vector (KS+; 2.9 kb) giving pBG120 (4.1 kb). The tyrosinase gene was removed from pBG120 with a XhoI/Smal cut and XhoI linkers were added to the SmaI end. This tyrosinase gene with XhoI ends was inserted into the XhoI site of the Stratagene bluescript vector (KS+; 2.9 kb) to give pBG130 (4.1 kb). Since there is an unwanted PstI site carried over in a poly-linker region, the pBG130 was modified by removing some of the non-coding bases and the poly-linker region. This was performed using a HindIII/SacI digest to release the tyrosinase from pBG130, then treating the 1.2 kb fragment with ExoIII to digest approximately 200 bases from the HindIII 3' end. After blunting the ends with T₄ DNA Polymerase, XhoI linkers were added and the 1.0 kb fragment was inserted into the Stratagene bluescript vector (Ks+; 2.9 kb) giving pBG132. The 1.0 kb tyrosinase gene was inserted into p803 (6.3 kb), which is a 3.6 kb subclone of the TMV plasmid, pS3-28 (available from W. O. Dawson, University of California, Riverside), in pUC19, giving plasmids pBG21 and pBG22 (depending on the direction of the tyrosinase gene; 7.3 kb each). The plasmid S3-28 (11.1 kb) is a clone of TMV that has had the coat protein gene removed with an XhoI site at that position. Dawson, W. O. et al., Phytopathology 78, 783 (1988). A NcoI/EcoRV cut of the pBG21 and pBG22 released a 2.4 kb fragment containing the tyrosinase gene. This fragment replaced a 1.4 kb piece in pS3-28 that had been removed. The resulting plasmids, pBG23 and pBG24 (depending on the direction of the tyrosinase gene; 12.1 kb each) were the final DNA constructions that were used to make infectious RNA that were introduced into tobacco plants to introduce a mRNA for tyrosinase. This mRNA can be detected using northern blot procedures.

### EXAMPLE 2

### Preparation of Chimeric TMV DNA Comprising GUS Gene in Place of the Coat Protein Gene

The 1.8 kb GUS gene was derived from the pRAJ275 (4.5 kb; Clontech Laboratories) using a EcoRI/NcoI cut. The sticky ends were filled in using the Klenow fragment. XhoI linkers were added and the fragment was inserted into the Stratagene bluescript vector (KS+), which is 2.9 kb in size. The resulting plasmid was maintained as pBG150 (4.7 kb). Using the techniques of Example 1, this GUS gene with the XhoI linkers was moved into the p803, giving pBG25 and pBG26 (depending on the direction of the GUS gene; 8.1 kb each). A SalI/NcoI cut of the pBG25 and pBG26 released a 3.8 kb fragment containing the GUS gene. These fragments replaced a 2.0 kb piece in pS3-28 that had been removed. The resulting plasmids, pBG27 and pBG28 (depending on the direction of the GUS gene; 12.9 kb each) were the final DNA constructions that were used to make infectious RNA that were introduced into tobacco plants to introduce a mRNA for the GUS gene. This mRNA can be detected using northern blot procedures. The activity of the GUS enzyme is also detectable.

### EXAMPLE 3

### Preparation of Chimeric TMV DNA Comprising GUS/Coat Protein Gene in Place of the TMV Coat Protein Gene

The 1.8 kb GUS gene from pBG150 (4.7 kbk), described in Example 2 above, was used for this construction. A Pst1/NcoI cut of p35-5 released a 0.7 kb fragment that contains a portion of the 3' end and a portion of the 5' end of the TMV coat protein gene. The plasmid p35-5 (11.3 kb) is a clone of TMV that has had most of the coat protein gene removed and contains an XhoI site at the site where the internal portion of the coat protein gene is removed. Dawson, W. O. et al., Phytopathology 78, 783 (1988). This 0.7 kb fragment replaced a 0.5 kb piece in p803 that had been removed, giving pBG29 (6.5 kb). The 1.8 kb GUS gene was inserted into the XhoI site of pBG29, giving pBG31 and pBG32 (depending on the direction of the GUS/coat protein fusion gene; 8.3 kb each). A SalI/NcoI cut of the pBG31 and pBG32 released a 4.0 kb fragment containing the GUS/coat protein fusion gene. These fragments replaced a 2.0 kb piece in pS3-28 that had been removed. The resulting plasmids, pBG33 and pBG34 (depending on the direction of the GUS/coat protein fusion gene; 13.1 kb each) were the final DNA constructions that were used to make infectious RNA. pBG33, pBG34 and pS3-28 (as control) are transcribed into RNA which is used to infect tobacco plants by rubbing on the plants the RNA, an abrasive and a RNase inhibitor. Spots are noted on the plants indicating a successful infection. The infected plant tissue is macerated and fluorescence examined. Tissue infected with pS3-28 did not fluoresce whereas tissue infected with either pBG23 or pBG34 did fluoresce.

### EXAMPLE 4

### Preparation of a Non-Transmissible TMV Nucleotide Sequence

A full-length DNA copy of the TMV genome is prepared and inserted into the Pst I site of pBR322 as described by Dawson, W.O. et al., Proc. Nat. Acad. Sci. USA 83, 1832 (1986). The viral coat protein gene is located at position 5711 of the TMV genome adjacent the 30k protein gene. The vector containing the DNA copy of the TMV genome is digested with the appropriate restriction enzymes and exonucleases to delete the coat protein coding sequence. For example, the coat protein coding sequence is removed by a partial digestion with ClaI and NsiI, followed by relegation to reattach the 3'-tail of the virus. Alternatively, the vector is cut at the 3' end of the viral nucleic acid. The viral DNA is removed by digestion with Bal31 or exonuclease III up through the start codon of the coat protein coding sequence. A synthetic DNA sequence containing the sequence of the viral 3'-tail is then ligated to the remaining 5'-end. The deletion of the coding sequence for the viral coat protein is confirmed by isolating TMV RNA and using it to infect tobacco plants. The isolated TMV RNA is found to be non-infective, i.e. biologically contained, under natural conditions.

### EXAMPLE 5

### Preparation of a Non-Transmissible OMV Nucleotide Sequence

A full-length DNA copy of the OMV genome is prepared as described by Dawson, W.O. et al., (1986), supra. The vector containing the DNA copy of the OMV genome is digested with the appropriate restriction enzymes or suitable exonucleases such as described in Example 4 to delete the coat protein coding sequence. The deletion of the coding sequence for the viral coat protein is confirmed by isolating OMV RNA and using it to infect germinating barley plants. The isolated OMV RNA is found to be biologically contained under natural conditions.

### EXAMPLE 6

### Preparation of a Non-Transmissible RNV Nucleotide Sequence

A full-length DNA copy of the RNV genome is prepared as described by Dawson, W.O. et al., (1986), supra. The vector containing the DNA copy of the RNV genome is digested with the appropriate restriction enzymes or suitable exonucleases such as described in Example 4 to delete the coat protein coding sequence. The deletion of the coding sequence for the viral coat protein is confirmed by isolating RNV RNA and using it to infect germinating barley plants. The isolated RNV RNA is found to be non-infective under natural conditions

### EXAMPLE 7

### Preparation of a Non-Transmissible PVY or PVX Nucleotide Sequence

A full-length DNA copy of the PVY or PVX genome is prepared as described by Dawson, W.O. et al., (1986), supra. The vector containing the DNA copy of the PVY or PVX genome is digested with the appropriate restriction enzymes or suitable exonucleases such as described in Example 4 to delete the coat protein coding sequence. The deletion of the coding sequence for the viral coat protein is confirmed by isolating PVY or PVX RNA and using it to infect potato plants. The isolated PVY or PVX RNA is found to be biologically contained under natural conditions.

### EXAMPLE 8

### Preparation of Chimeric Nucleotide Sequence Containing the Tyrosinase Coding Sequence

The coding sequence for tyrosinase is isolated from Streptococcus antibioticus, by digestion with BclI followed by 5'-exonuclease digestion to the start codon. Alternatively, a restriction site is engineered adjacent the start codon of the tyrosinase coding sequence by site-directed oligonucleotide mutagenesis. Digestion with the appropriate restriction enzyme yields the coding sequence for tyrosinase. The fragment containing the tyrosinase coding sequence is isolated and cloned adjacent the promoter of the viral coat protein gene in the vectors prepared in Examples 4, 5 and 6.

### EXAMPLE 9

### Preparation of Virus Containing Tyrosinase

A lambda P_{R} promoter is attached to the chimeric nucleotide sequence of Example 8 in accordance with the technique described in Dawson, W.O. et al., (186), supra. The resulting vector is used to transform E. coli, the production cell in this instance.

A second vector is prepared by inserting the viral coat protein coding sequence, isolated in Examples 4, 5 or 6, adjacent the lac promoter in the vector pBR322. This vector is used to transform the production cells having a vector with the corresponding compatible chimeric nucleotide sequence. The production cells are grown and the resultant viruses are isolated. Alternatively, the second vector is used to transform a second strain of E. coli which produces the coat protein. The coat protein and viral vector are then combined to form the virus.

### EXAMPLE 10

### Production of Melanin

The viruses isolated in Example 9 are used to infect tobacco plants (viruses based on TMV) or germinating barley plants (viruses based on OMV or RNV). The infected plants are grown under normal growth conditions. The plants produce tyrosinase which reacts with the components present in the plant tissue to produce intermediates which are converted to melanin within the plant tissue or after the plant tissue is lyzed. The melanin is isolated by conventional techniques.

### EXAMPLE 11

### Preparation of a Chimeric Nucleotide Sequence Containing a Beta Cyclodextrin Glucanotransferase Coding Sequence

The coding sequence for beta cyclodextrin glucotransferase is isolated from alkalophilic Bacillus sp. strain No. 38-2 in the following manner.

The chromosomal DNA of strain No. 38-2 (Hanamoto, T. et al., Agric. Biol. Chem. 51, 2019 (1987)) is partially cleaved with Sau3AI and the fragments are ligated in BamHI-digested pBR322. A transformant carrying plasmid pCS115, which contains a 3.2 kb DNA fragment from the genome of the producing strain, has the CGT activity. The CGT produced by this transformant gives one line of precipitation which fuses completely with that for the No. 38-2 CGT by an Ouchterlony double-diffusion test. The nucleotide sequence of the fragment is found by the dideoxy chain termination reaction (Sanger, F. et al., Proc. Nat. Acad. Sci. USA 74, 5463 (1977)) using pUC19, and the exonuclease deletion method (Henikoff, S., Gene 28, 351 (1984)). The nucleotide sequence of the fragment shows a single open reading frame corresponding to the CGT gene. A protein with a molecular mass of 66 kDal could be translated from this open reading frame of 1758 bp. For the detailed nucleotide sequence, see Hanamoto, T. et al., supra.

The sequence of the N-terminal amino acids of the extracellular form of CGT is found with a peptide sequencer. NH₂-Ala-Pro-Asp-Thr-Ser-Val-Ser-Asn-Lys-Gln-Asn-Phe-Ser-Thr-Asp-Val-Ile is identical to that deduced from the DNA sequence (residues 1 to 17). This result suggests that 27 amino acid residues (residues -27 to - 1) represent a signal peptide which is removed during secretion of CGT. The molecular weight of the matured CGT calculated from the DNA sequence is 63,318.

A probe is prepared based on a portion of the amino acid sequence of cyclodextrin glucanotransferase and used to isolate the coding sequence for this enzyme. Alternatively, the beta cyclodextrin glucotransferase coding sequence is isolated following reverse transcription. The fragment containing the coding sequence is isolated and cloned adjacent the promoter of the viral coat protein gene in the vectors prepared in Examples 5, 6 or 7.

### EXAMPLE 12

### Preparation of Virus Containing a Cyclodextrin Glucanotransferase Coding Sequence

A lambda P_{R} promoter is attached to the chimeric nucleotide sequence of Example 8 in accordance with the technique described in Dawson, W.O. et al., (1986), supra. The resulting vector is used to transform E. coli, the production cell in this instance.

A second vector is prepared by inserting the viral coat protein coding sequence, isolated in Examples 5, 6 or 7, adjacent the lac promoter in the vector pBR322. This vector is used to transform the production cells having a vector with the corresponding compatible chimeric nucleotide sequence. The production cells are grown and the resultant viruses are isolated.

### EXAMPLE 13

### Production of Cyclodextrin

The viruses isolated in Example 12 are used to infect corn plants (viruses basad on OMV or RNV) or potato plants (viruses based on PVY or PVX). The infected plants are grown under normal growth conditions. The plants produce cyclodextrin glucotransferase which catalyzes the conversion of starch to cyclodextrin in the plant tissue. The cyclodextrin is isolated by conventional techniques.

### EXAMPLE 14

### Preparation of a Chimeric Nucleotide Sequence of TMV and Chloramphenicol Acetyltransferase

A non-transmissible TMV nucleotide sequence (pTMVS3-28) is prepared as described in Example 4. A chimeric nucleotide sequence containing the chloramphenicol acetyltransferase (CAT) gene substituted for the previously removed coat protein gene (S3-CAT-28) is then prepared.

The CAT gene is removed from pCM1 (Pharmacia) with Sal I and ligated into the nucleotide sequence from Example 4 which has been cleaved by Xho I. This construction produces pTMVS3-CAT-28 from which the mutant cp S3-CAT-28 is transcribed. Correct sequence and orientation is confirmed by sequencing as described by Sagursky, R.J. et al., Gene Anal. Technol. 2, 89 (1985).

This chimeric DNA sequence is then transcribed using the PM promoter (Ahlquist, P. et al., Mol. Cell Biol. 4, 2876 (1984)) and RNA polymerase of E. coli (Dawson, W.O. et al., Proc. Nat. Acad. Sci. USA, 83, 1832 (1986)) to produce infectious chimeric RNA. The infectivity is assayed by grinding leaves in cold 1% sodium pyrophosphate buffer, pH9.0, plus 1% bentonite and 1% Celite with immediate inoculation. Alternatively, the infectivity may be assayed using phenol-extracted RNA in the same buffer (Dawson, W.O., Virology, 73, 319 (1976)).

The chimeric RNA is found to replicate effectively in tobacco leaves. The RNA can be propagated serially from plant to plant or may be stored after phenol extraction. However, no virions are produced in the infected plants.

The chimeric RNA fails to produce a band during SDS-PAGE that can be positively identified as chloramphenicol acetyltransferase. However, functional enzyme activity can be detected in plant extracts that acetylated ¹⁴C-chloramphenicol with acetyl CoA.

### EXAMPLE 15

### Preparation of a Virus Containing Chloramphenicol Acetyltransferase

A lambda P_{R} promoter is attached to the chimeric nucleotide sequence of Example 14 in accordance with the technique described by Dawson, W. O. et al., Proc. Nat. Acad. Sci. U.S.A. 83, 1832 (1986). The resulting vector is used to transform the production cell, E. coli

A second vector is prepared by inserting the viral coat protein coding sequence (isolated in Example 4), adjacent the lac promoter in the vector pBR322. This vector is used to transform the E. coli production cells having the first vector with the chimeric nucleotide sequence. The production cells are grown, and the resultant viruses are isolated. Alternatively, the second vector is used to transform a second strain of E. coli which produces the coat protein. The coat protein and viral vectors are then combined to form the virus.

### EXAMPLE 16

### Production of Chloramphenicol Acetyltransferase

The viruses isolated in Example 15 are used to infect tobacco plants. The infected plants are grown under normal growth conditions. The plants produce the enzyme, chloramphenicol. acetyltransferase, which is isolated from the plants using conventional techniques.

### EXAMPLE 17

### Preparation of a Non-Infective Eastern Equine Encephalomyelitis Virus Nucleotide Sequence

A full-length cDNA copy of the Eastern Equine Encephalomyelitis Virus (EEEV) genome is prepared and inserted into the PstI site of pUC18 as described by Chang, G-J. J. et al., J. Gen. Virol., 68, 2129 (1987). The sequence for the viral coat protein and its adjacent E1 and E2 glycoprotein transmissibility factors are located on the region corresponding to the 26S RNA region. The vector containing the cDNA copy of the EEEV genome is digested with the appropriate restriction enzymes and exonucleases to delete the coding sequence of the coat protein and the E1 and E2 proteins (structural protein coding sequence).

For example, the structural protein coding sequence is removed by partial digestion with MboI, followed by religation to remove a vital portion of the structural gene. Alternatively, the vector is cut at the 3' end of the viral structural gene. The viral DNA is sequentially removed by digestion with Bal31 or Micrococcal S1 nuclease up through the start codon of the structural protein sequence. The DNA sequence containing the sequence of the viral 3'-tail is then ligated to the remaining 5'-end. The deletion of the coding sequence for the structural proteins is confirmed by isolating EEEV RNA and using it to infect an equine cell culture. The isolated EEEV RNA is found to be non-infective under natural conditions.

Alternatively only the coding sequence for the coat protein is deleted and the sequence for the E1 and E2 glycoproteins remain in the vector containing the cDNA copy of the EEEV genome. In this case, the coat protein coding sequence is removed by partial digestion with MboI followed by religation to reattach the 3'-tail of the virus. This will remove a vital portion of the coat protein gene.

A second alternative method for removing only the coat protein sequence is to cut the vector at the 3 '-end of the viral coat protein gene. The viral DNA is removed by digestion with Bal31 or Micrococcal S1 nuclease up through the start codon of the coat protein sequence. The synthetic DNA sequence containing the sequence of the 3'-tail is then ligated to the remaining 5'-end.

The deletion of the coding sequence for the coat protein is confirmed by isolating EEEV RNA and using it to infect an equine cell culture. The isolated EEEV RNA is found to be non-infective under natural conditions.

### EXAMPLE 18

### Preparation of a Non-Transmissible Sindbis Virus Nucleotide Sequence

A full-length cNDA copy of the Sindbis virus genome is prepared and inserted into the SmaI site of a plasmid derived from pBR322 as described by Lindquist, B.H. et al., Virology, 151, 10 (1986). The sequence for the viral coat protein and the adjacent E1 and E2 glycoprotein transmissibility factors are located on the region corresponding to the 26S RNA region. The vector containing the cDNA copy of the Sindbis virus genome is digested with the appropriate restriction enzymes and exonucleases to delete the coding sequence for the structural proteins. For example, the structural protein coding sequence is removed by partial digestion with BinI, followed by religation to remove a vital portion of the structural gene. Alternatively, the vector is cut at the 3' end of the viral nucleic acid. The viral DNA is removed by digestion with Bal31 or Micrococcal S1 nuclease up through the start codon of the structural protein sequence. The synthetic DNA sequence containing the sequence of the viral 3'-tail is then ligated to the remaining 5'-end. The deletion of the coding sequence for the structural proteins is confirmed by isolating Sindibis RNA and using it to infect an avian cell culture. The isolated Sindbis RNA is found to be non-infective under natural conditions.

Alternatively only the coding sequence for the coat protein is deleted and the sequence for the E1 and E2 glycoproteins remain in the vector containing the cDNA copy of the Sindbis genome. In this case, the coat protein coding sequence is removed by partial digestion with AflII followed by religation to reattach the 3'-tail of the virus.

A second alternative method for removing only the coat protein sequence is to cut the vector at the 3'-end of the viral nucleic acid. The viral DNA is removed by digestion with Bal31 or Micrococcal S1 nuclease up through the start codon of the coat protein sequence (the same start codon as for the sequence for all the structural proteins). The synthetic DNA sequence containing the sequence of the 3'-tail is then ligated to the remaining 5'-end.

The deletion of the coding sequence for the coat protein is confirmed by isolating Sindibis RNA and using it to infect an avian cell culture. The isolated Sindbis RNA is found to be non-infective under natural conditions.

### EXAMPLE 19

### Preparation of a Non-Transmissible Western Equine Encephalomyelitis Virus Nucleotide Sequence

A full-length cDNA copy of the Western Equine Encephalomyelitis Virus (WEEV) genome is prepared as described by Hahn, C.S. et al., Proc. Natl. Acad. Sci. USA 85, 5997 (1988). The sequence for the viral coat protein and its adjacent E1 and E2 glycoprotein transmissibility factors are located on the region corresponding to the 26S RNA region. The vector containing the cDNA copy of the WEEV genome is digested with the appropriate restriction enzymes and exonucleases to delete the coding sequence of the coat protein and the E1 and E2 proteins (structural protein coding sequence).

For example, the structural protein coding sequence is removed by partial digestion with NacI, followed by religation to remove a vital portion of the structural protein sequence. Alternatively, the vector is cut at the 3' end of the structural protein DNA sequence. The viral DNA is removed by digestion with Bal31 or Micrococcal S1 nuclease up through the start codon of the structural protein sequence. The DNA sequence of the viral 3'-tail is then ligated to the remaining 5'-end. The deletion of the coding sequence for the structural proteins is confirmed by isolating WEEV RNA and using it to infect a Vero cell culture. The isolated WEEV RNA is found to be non-infective under natural conditions.

Alternatively only the coding sequence for the coat protein is deleted and the sequence for the E1 and E2 glycoproteins-remain in the vector containing the cDNA copy of the WEEV genome. In this case, the coat protein coding sequence is removed by partial digestion with HgiAI followed by religation to reattach the 3'-tail of the virus.

A second alternative method for removing only the coat protein sequence is to cut the vector at the 3'-end of The viral coat protein sequence. The viral DNA is removed by digestion with Bal31 or Micrococcal S1 nuclease up through the a vital portion of the coat protein sequence. The DNA sequence containing the sequence of the 3'-tail is then ligated to the remaining 5'-end.

The deletion of the coding sequence for the coat protein is confirmed by isolating WEEV RNA and using it to infect a Vero cell culture. The isolated WEEV RNA is found to be non-infective, i.e. biologically contained, under natural conditions.

### EXAMPLE 20

### Preparation of Chimeric Nucleotide Sequence Containing the Tyrosinase Coding Sequence

The coding sequence for tyrosinase is isolated as described in Example 8, and cloned adjacent the promoter of the viral coat protein gene in the vectors prepared in Examples 17, 18 and 19.

### EXAMPLE 21

### Preparation of Virus Containing Tyrosinase

A promoter is attached to the chimeric nucleotide sequence of Example 20 in accordance with conventional techniques. The resulting vector is used to transform the production cell which is yeast in this instance.

A second vector is prepared by inserting the viral structural protein coding sequence, isolated in Examples 17, 18 and 19, adjacent the ADCI promoter in the vector pAH5 (Ammerer, G., Meth. Enzymol. 101, 192 (1983)). This vector is used to transform the production cells having a vector with the compatible chimeric nucleotide sequence. The production cells are grown and the resultant viruses are isolated. Alternatively, the second vector is used to transform a second strain of yeast which produces the structural proteins. The structural proteins and the viral vector are then combined to form the virus.

### EXAMPLE 22

### Preparation of Melanin In Vitro

The viruses isolated in Example 21, made by the combination of chimeric nucleotide sequence and coat protein are used to infect equine cell cultures (viruses based on EEEV and WEEV) or avian cell cultures (viruses based on Sindbis virus). The infected cell cultures are grown under normal cell culture growth conditions. The cells produce tyrosinase which reacts with the components present in the cells to produce intermediates which are then converted to melanin. The melanin is isolated by conventional techniques.

### EXAMPLE 23

### Preparation of Melanin In Vivo

The viruses isolated in Example 21, made by the combination of chimeric nucleotide sequence and structural proteins (coat protein, E1 glycoprotein and E2 glycoprotein) are used to infect horses (viruses based on EEEV and WEEV) or chickens (viruses based on Sindbis virus). The infected animals are maintained under normal conditions (i.e. feeding, exercise, sleep etc.) The animals produce tyrosinase which reacts with components present in the animals to produce intermediates which are then converted to melanin. The melanin is isolated by conventional techniques.

### EXAMPLE 24

### Preparation of a Chimeric Nucleotide Sequence Containing Human Tissue Plasminogen Activator (t-PA) Coding Sequence

The coding sequence for human tissue plasminogen activator is isolated from plasmid pt-PAtrp12, ATCC No. 40404 (U.S. Patent 4,766,075) and cloned adjacent the promoter of the viral coat protein gene in the vectors prepared in Examples 17, 18 or 19.

### EXAMPLE 25

### Preparation of Virus Containing a Coding Sequence for Human t-PA

A virus containing the coding sequence for human t-PA is prepared in accordance with the procedures described in Example 21.

### EXAMPLE 26

### Preparation of Human t-PA In Vitro

The viruses isolated in Example 25, made by the combination of chimeric nucleotide sequence and coat protein are used to infect equine cell cultures (viruses based on EEEV and WEEV) or avian cell cultures (viruses based on Sindbis virus). The infected cell cultures are grown under normal cell culture growth conditions. The cells produce human t-PA which is isolated by conventional techniques.

### EXAMPLE 27

### Preparation of Human t-PA In Vivo

The viruses isolated in Example 25, made by the combination of chimeric nucleotide sequence and structural proteins (coat protein, E1 glycoprotein and E2 glycoprotein) are used to infect horses (viruses based on EEEV and WEEV) or chickens (viruses based on Sindbis virus). The infected animals are maintained under normal conditions (i.e. feeding, exercise, sleep etc.) The animals produce human t-PA which is isolated by conventional techniques

### EXAMPLE 28

### Preparation of a Non-Infective Human Rhinovirus 2 Nucleotide Sequence

A full length cDNA copy of the human rhinovirus 2 (HRV2) genome is prepared, and inserted into the PstI site of plasmid pUC9 as described by Skern, T. et al., Nucleic Acids Res., 13, 2111 (1985). The nucleotide Sequence for the viral coat protein VP1 is located at position 2644 of the genome. The vector containing the DNA copy of the HRV2 genome is digested with the appropriate restriction enzymes and exonucleases to delete the coat protein coding sequence.

For example, the coat protein coding sequence is removed by partial digestion with an appropriate restriction endonuclease, followed by religation to remove a vital portion of the coat protein sequence. Alternatively, the vector is cut at the 3'-end of the viral coat protein gene. The viral DNA is removed by digestion with Bal31 or Micrococcal S1 nuclease up through the start codon (promoter) of the coat protein Sequence. The synthetic DNA sequence containing the sequence of the viral 3'-tail is then ligated to the remaining 5'-end. The deletion of the coding sequence for the coat protein is confirmed by isolating HRV2 RNA and using it to infect a human cell culture. The isolated HRV2 RNA is found to the non-infective under natural conditions.

### EXAMPLE 29

### Preparation of Chimeric Nucleotide Sequence Containing the Tyrosinase Coding Sequence

The coding sequence for tyrosinase is isolated as described in Example 8, and cloned adjacent the promoter of the viral coat protein gene in the vectors prepared in Example 28.

### EXAMPLE 30

### Preparation of Virus Containing Tyrosinase

A virus based on HRV2 containing a tyrosinase coding sequence is prepared as described in Example 21 using the starting materials produced in Examples 28 and 29.

### EXAMPLE 31

### Preparation of Melanin

The viruses isolated in Example 30 are used to infect human cell cultures. The infected cells produce tyrosinase which reacts with the components present in the human cells to produce intermediates which are then converted to melanin in the cells. The melanin is isolated by conventional techniques.

### EXAMPLE 32

### Preparation of a Chimeric Nucleotide Sequence Containing a Human t-PA Coding Sequence

The coding sequence for human t-PA is isolated as described in Example 24, and cloned adjacent the promoter of the viral coat protein gene in the vectors prepared in Example 28.

### EXAMPLE 33

### Preparation of Virus Containing a Coding Sequence for Human t-PA

A virus containing the coding sequence for human t-PA is prepared in accordance with the procedures of Example 30.

### EXAMPLE 34

### Preparation of Human t-PA

The viruses isolated in Example 33 are used to infect human cell cultures. The infected cells are grown under normal growth conditions. The cells produce human t-PA which is isolated by conventional techniques.

### EXAMPLE 35

### Preparation of a Non-Infective Poliovirus Type 2 Nucleotide Sequence

A full-length cDNA copy of the poliovirus type 2 (PV2) genome is prepared, and inserted into the HindIII site of plasmid pBR322 as described by Toyoda, H. et al., J. Mol. Biol., 174, 561 (1984). The nucleotide sequence of the viral coat protein VP1 is located at position 2499 of the genome. The vector containing the DNA copy of the PV2 genome is digested with the appropriate restriction enzymes and exonucleases to delete the coat protein coding sequence. It is important that the protease digestion sequences are left intact.

For example, a part of the coat protein coding sequence is removed by partial digestion with an appropriate restriction endonuclease, followed by religation to reattach the 3'-tail of the virus. The deletion of the coding sequence for the coat protein is confirmed by isolating PV2 RNA and using it to infect spinner-cultured HeLa S3 cells. The isolated PV2 RNA is found to be non-infective,i.e. biologically contained, under natural conditions.

### EXAMPLE 36

### Preparation of Chimeric Nucleotide Sequence Containing the Tyrosinase Coding Sequence

The coding sequence for tyrosinase is isolated as described in Example 8, and cloned adjacent the promoter of the viral coat protein gene in the vectors prepared in Example 35.

### EXAMPLE 37

### Preparation of Virus Containing Tyrosinase

A virus based on PV2 containing a coding sequence for tyrosinase is prepared as described in Example 21 using the starting materials produced in Example 35 and 36.

### EXAMPLE 38

### Preparation of Melanin

The viruses isolated in Example 37 are used to infect spinner-cultured HeLa S3 cells. The infected cells produce tyrosinase which reacts with the components present in the cells to produce intermediates which are then converted to melanin in the cells. The melanin is isolated by conventional techniques.

### EXAMPLE 39

### Preparation of a Chimeric Nucleotide Sequence Containing a Human t-PA Coding Sequence

The coding sequence for human t-PA is isolated as described in Example 24, and cloned adjacent the promoter of the viral coat protein gene in the vectors prepared in Example 35.

### EXAMPLE 40

### Preparation of Virus Containing Human t-PA Coding Sequence

A virus containing the coding sequence for human t-PA prepared in accordance with the procedures of Example 37.

### EXAMPLE 41

### Preparation of Human t-PA

The viruses of Example 40 are used to infect spinner-cultured HeLa S3 cells. The infected cells are grown under normal growth conditions. The cells produce human t-PA which is isolated by conventional techniques.

### EXAMPLE 42

### Preparation of a Non-Infective Simian Virus 40 Nucleotide Sequence

A full-length cDNA copy of the Simian virus 40 (SV40) genome is prepared, and inserted into the AccI site of plasmid pCW18 as described by Wychowski, C. et al., J. Virol. 61, 3862 (1987). The nucleotide sequence of the viral coat protein VP1 is located between position 1488 and 2574 of the genome. The vector containing the DNA copy of the SV40 genome is digested with the appropriate restriction enzymes and exonucleases to delete the coat protein coding sequence.

For example, the VP1 coat protein coding sequence is removed by partial digestion with BamHI nuclease, and then treated with EcoRI, filled in with Klenow enzyme and recircularized. The deletion of the coding sequence for the coat protein VP1 is confined by isolating SV40 RNA and using it to infect simian cell cultures. The isolated SV40 RNA is found to be non-infective, i.e. biologically.contained, under natural conditions.

### EXAMPLE 43

### Preparation of Chimeric Nucleotide Sequence Containing the Tyrosinase Coding Sequence

The coding sequence for tyrosinase is isolated as described in Example 8, and cloned adjacent the promoter of the viral coat protein gene in the vectors prepared in Example 42.

### EXAMPLE 44

### Preparation of Virus Containing Tyrosinase

A virus containing a tyrosinase coding sequence and based on SV40 is prepared as described in Example 21. The chimeric nucleotide sequence of Example 43 and the coat protein coding sequence isolated in Example 42 are Utilized.

### EXAMPLE 45

### Preparation of Melanin

The viruses isolated in Example 44 are used to infect simian cell cultures. The infected cells produce tyrosinase which reacts with the components present in the cells to produce intermediates which are then converted to melanin in the cells. The melanin is isolated by conventional techniques.

### EXAMPLE 46

### Preparation of a Chimeric Nucleotide Sequence Containing a Human t-PA Coding Sequence

The coding sequence for cyclodextrin glucotransferase is isolated as described in Example 24, and cloned adjacent the promoter of the viral coat protein gene in the vectors prepared in Example 42.

### EXAMPLE 47

### Preparation of Virus Containing Human t-PA Coding Sequence

A virus containing the coding sequence for human t-PA is prepared in accordance with the procedures of Example 44.

### EXAMPLE 48

### Preparation of Human t-PA

The viruses of Example 47 are used to infect simian cell cultures. The infected cells are grown under normal growth conditions. The cells produce human t-PA which is isolated by conventional techniques.

### EXAMPLE 49

### Transformation of Nicotiana Tobacum with Capsid Protein Gene

The capsid protein of tobacco mosaic virus (TMV) is encoded by a nucleotide sequence between 5712 and 6190. A 3'-untranslated region of the RNA genome extends to nucleotide 6395. A double stranded (ds) complementary DNA (cDNA) of the cistron containing nucleotide 5701 to 6395 and coding for capsid protein, is generated from TMV-RNA using reverse transcriptase. The transcriptase contains an oligonucleotide primer which is complementary to an oligonucleotide sequence at the 3' end of the viral RNA and contains a BamHI site.

A Klenow fragment of DNA polymerase is used to synthesize the second DNA-strand. The HindIII-BamHI fragment of cDNA is cloned into the plasmid pUC9 (Vieira, J. et al., Gene 19, 259 (1982)). The resulting plasmid is digested with AlaIII at nucleotide 5707 (Goelet, P. et al., Proc. Natl. Acad. Sci. USA 79, 5818 (1982)) and with BamHI to remove the sequence for capsid protein. This fragment is ligated into the vector pMON237.

pMON237 is a derivative of pMON200 (Horsch, R.B. et al., Science 227, 1229 (1985)). This vector contains a 19S CaMV promoter, a polylinker sequence and the sequence for nopaline synthese (NOS) at the 3' end. Ligation occurs at the XbaI site which is made blunt, and at the BamHI site. The capsid protein coding sequence is removed from the plasmid by digestion with XbaI and BamHI and given a BglII site at the 5' end and an EcoRI site at the 3' end by transfer to a suitable plasmid.

The BglII-EcoRI fragment is transferred into the expression cassette vector pMON316 between the CaMV 35S promoter and the NOS 3' untranslated region (Rogers, S.G. et al., in BioTechnology in Plant Science: Relevance to Agriculture in the Nineteen Eighties, M. Zaitlin, P. Day, A. Hollaender, Eds., Academic Press, N.Y., p. 219 (1986). The resulting chimeric gene contains the 35S promoter from CaMV and a polyadenylation signal from the NOS gene. The resulting plasmid is mated into Agrobacterium tumefaciens strain GV 3111 carrying the disarmed pTi BGS3-SE plasmid (Fraley, R.T. et al., Bio/Technology 3' 629 (1985)). A cointegrate plasmid results by recombination between LIH regions.

Transformed A. tumefaciens are selected for antibiotic resistance. Selected colonies are used to transform N. tobacum leaf disks which are then regenerated into whole plants (Horsch, R.B. et al., supra; Abel, P.P. et al., Science 232, 738 (1986)). A. tumefaciens transformed with the sequence for TMV capsid protein is used as a production cell. Likewise tobacco cells that have been transformed with the sequence for TMV capsid protein are used as production cells for producing capsid protein monomers or multimers. Capsid protein numbers and timing of production can be controlled by methods well known in the arts (Maniatis, T. et al., supra). Proper selection of a temperature sensitive repressor and a strong promoter results in high copy numbers of plasmids and high translation rates only when desired.

### EXAMPLE 50

### Production of Encapsidated Virus Particles

Protoplasts of transformed tobacco cells obtained in the Example 49 are resuspended in a 0.5 M mannitol solution containing 12-30 nM MgCl₂. A heat shock of 45°C for five minutes is given. The protoplasts are distributed in aliquots for transformation in centrifuge tubes, 0.3 ml of suspended protoplasts per tube. During the next 10 minutes the following are added. Plasmids obtained in Example 1 containing cDMA coding for the TMV genome but missing the gene for capsid protein, and which contains a chimeric gene construct; and polyethylene glycol (PEG) solution (MW 6000, 40% (w/v) containing 0.1M Ca(NO₃)₂ and 0.4M mannitol; pH 8-9 with KOH) to give a final concentration of 20% PEG. The aliquots are incubated for 30 minutes with occasional gentle shaking, and then the protoplasts are placed in petri dishes (0.3 ml original protoplast suspension per 6 cm diameter dish) and cultured. Encapsidated virus particles are isolated from the protoplasts using conventional techniques.

Alternatively, the transformed plants produced in Example 49 are infected with virus nucleic acid, e.g. a chimeric nucleotide sequence of Example 3. The nucleic acid is replicated and coat protein is produced which encapsidates the nucleic acid. The encapsidated virus particles are isolated using conventional techniques.

### EXAMPLE 51

### Transformation of Nicotiana Tobacum with Replicase Gene

Two replicase subunits of tobacco mosaic virus (TMV) are encoded by a nucleotide sequence between 70 and 4919. The nucleotide sequence for the 130-kD protein terminates at an amber codon which can be suppressed to produce a 180-kD protein. A double stranded (ds) complementary DNA (cDNA) of the cistron containing nucleotides 70 to 4919 and coding for replicase, is generated from TMV-RNA using reverse transcriptase.

The transcriptase contains an oligonucleotide primer which is complementary to an oligonucleotide sequence at the 3' end of the viral RNA and contains a BamHI site. A Klenow fragment of DNA polymerase is used to synthesize the second DNA-strand. The HindIII-BamHi fragment of cDNA is cloned into the plasmid pUC9 (Viera, J. et al., Gene 19, 259 (1982)). The resulting plasmid is digested with AlaIII at nucleotide 4919 (Goelet, P. et al., Proc. Natl. Acad. Sci. USA 79, 5818 (1982)) and with BamHI to remove the sequence for capsid protein.

This fragment is ligated into the vector pMON237. pMON237 is a derivative of pMON200 (Horsch, R.B. et al., Science 227, 1229 (1985)). This vector contains a 19S CaMV promoter, a polylinker sequence and the sequence for nopaline synthase (NOS) at the 3' end. Ligation occurs at the XbaI site which is made blunt, and at the BamHI site. The capsid protein coding sequence is removed from the plasmid by digestion with XbaI and BamHI and given a BglII site at the 5' end and EcoRI site at the 3' end by transfer to a suitable plasmid. The BglII-EcoRI fragment is transferred into the expression cassette vector, pMON316 between the CaMV 35S promoter and the NOS 3' untranslated region (Rogers, S.G. et al., in BioTechnology in Plant Science: Relevance to Agriculture in the Nineteen Eighties, M. Zaitlin, P. Day, A. Hollaender, Eds., Academic Press, N.Y., p. 219, 1986). The resulting chimeric gene contains the 35S promoter from CaMV and a polyadenylation signal from the NOS gene. The resulting plasmid is mated into Agrobacterium tumefaciens strain GV 3111 carrying the disarmed pTi BGS3-SE plasmid (Fraley, R.T. et al., Bio Technology 3, 629 (1985)). A cointegrate plasmid results by recombination between LIH regions. Transformed A. tumefaciens are selected for antibiotic resistance. Selected colonies are used to transform N. tobacum leaf disks which are then regenerated into whole plants (Horsch, R.B. et al., supra, Abel, P.P. et al., Science 232, 738 (1986)). The transformed hosts can be used as production cells or hosts for the infection with TMV based viruses. The stably incorporated replicase will function to enhance replication of the viral nucleic acid.

### EXAMPLE 52

### Transformation of Tobacco Cells with Transport Protein

A nucleotide sequence of TMV from nucleotide 4903 to 5709 encodes a 30-kD protein which is implicated in facilitating cell to cell movement of the virus. Leaf disk cells of N. tobacum are transformed by Agrobacterium carrying a cointegrate plasmid made by the method of Example 49. N. tobacum cells so transformed are used as host cells for encapsidated defective virus particles.

While the invention has been described in connection with specific embodiments thereof, it will be understood that it is capable of further modifications. This application is intended to cover any variations, uses or adaptations of the invention following, in general, the principles of the invention and including such departures from the present disclosure as come within known and customary practice within the art to which the invention pertains.

In the following passages especially preferred embodiments are described.

One preferred embodiment relates to a nucleotide sequence having substantial sequence homology to a viral nucleotide sequence which is capable of replication and is biologically contained. The viral nucleotide sequence may be a prokaryotic or eukaryotic nucleotide sequence. The viral nucleotide may be a plant viral nucleotide sequence. The above-mentioned nucleotide sequence my be selected from the group consisting of DNA, RNA or cDNA. It is further preferred that the viral nucleotide sequence lacks a biologically functional viral coat protein coding sequence and optionally further lacks a biologically functional viral transmissiblity factor coding sequence.

Another preferred embodiment relates to a chimeric nucleotide sequence comprising a first nucleotide sequence which has substantial sequence homology to a viral nucleotide sequence which is capable of replication and is biologically contained, and a second nucleotide sequence which is adjacent a viral promoter and which is a coding sequence capable of transcription in a host. The viral nucleotide sequence may be a prokaryotic viral nucleotide sequence or a eukaryotic viral nucleotide sequence. The viral nucleotide sequence may be a plant viral nucleotide sequence. The above-mentioned chimeric nucleotide sequence may be selected from the group consisting of DNA, RNA and cDNA. It is preferred that the viral nucleotide sequence lacks a biologically functional viral coat protein coding sequence and optionally further lacks a biologically functional viral transmissibility factor coding sequence. In the above-mentioned nucleotide sequence or the chimeric nucleotide sequence, the viral promoter may be a viral coat protein promoter.

Another preferred embodiment relates to a virus comprising a chimeric nucleotide sequence which comprises a first nucleotide sequence which has substantial sequence homology to a viral nucleotide sequence and which is capable of repliction and is biologically contained, and a second nucleotide sequence which is adjacent a viral promoter and which is a coding sequence capable of transcription in a host. The viral nucleotide sequence may be a prokaryotic viral nucleotide sequence or a eukaryotic viral nucleotide sequence. The viral nucleotide sequence may be a plant viral nucleotide sequence. It is preferred that the chimeric nucleotide sequence may be selected from the group consisting of DNA, RNA and cDNA. It is further preferred that the viral nucleotide sequence lacks a biologically functional viral coat protein coding sequence, and optionally further lacks a biologically functional viral transmissibility factor coding sequence. In the virus the viral promoter may be a viral coat protein promoter.

Another preferred embodiment relates to a vector comprising a chimeric nucleotide sequence adjacent a nucleotide sequence selected from a production cell promoter and an origin of replication compatible with said production cell; the chimeric nucleotide sequence comprising a first nucleotide sequence which has substantial sequence homology to a viral nucleotide sequence which is capable of replication and is biologically contained, and a second nucleotide sequence which is adjacent a viral promoter and which is a coding sequence capable of transcription in a host. The viral nucleotide sequence may be a prokaryotic viral nucleotide sequence or eukaryotic viral nucleotide sequence. It is preferred that the viral nucleotide sequence is a plant viral nucleotide sequence. In the vector it is preferred that the chimeric nucleotide sequence is selected from the group consisting of DNA, RNA and cDNA. It is further preferred that the viral nucleotide sequence lacks a biologically functional viral coat protein coding sequence, and optionally further lacks a biologically functional viral transmissibility factor coding sequence. In the vector the viral promoter may be a viral coat protein promoter.

Another preferred embodiment relates to a production cell having a vector which comprises a chimeric nucleotide sequence adjacent a nucleotide sequence selected from the group consisting of a production cell promoter and an origin of replication compatible with the production well, said chimeric nucleotide sequence comprising a first nucleotide sequence which has substantial sequence homology to a viral nucleotide sequence which is capable of replication and is biologically contained, and a second nucleotide sequence which is adjacent a viral promoter and which is a coding sequence capable of transcription in a host. The viral nucleotide sequence may be a prokaryotic viral nucleotide sequence or a eukaryotic viral nucleotide sequence. It is preferred that the viral nucleotide sequence is a plant viral nucleotide sequence. In the production cell the chimeric nucleotide sequence may be selected from the group consisting of DNA, RNA and cDNA. It is further preferred that the viral nucleotide sequence lacks a biologically functional viral coat protein coding sequence, and optionally further lacks a biologically functional viral transmissibility factor coding sequence. In the production cell the viral promoter may be a viral coat protein promoter. It is preferred that the production cell further has a second vector which comprises a viral coat protein coding sequence adjacent a production cell promoter, the viral coat protein being compatible with the first nucleotide sequence and being biologically functional. It is further preferred that the production cell has been stably transformed to contain a coding sequence for a viral replicase useful for the replication of the chimeric nucleotide sequence. It is further preferred that the production cell has been stably transformed to contain a coding sequence for a viral coat protein compatible with the chimeric nucleotide sequence. It is further preferred that the production cell has been further stably transformed to contain a coding sequence for a viral replicase useful for the replication of the chimeric nucleotide sequence. It is further preferred that the production cell has been stably transformed to contain a coding sequence for a viral transmissibility factor compatible with the chimeric nucleotide sequence. It is further preferred that the production cell has been further stably transformed to contain a coding sequence for a replicase useful for the replication of the chimeric nucleotide sequence. It is further preferred that the production cell has been stably transformed to contain a coding sequence for a viral coat protein and a viral transmissibility factor compatible with the chimeric nucleotide sequence. It is further preferred that the production cell has been stably transformed to contain coding sequences for a replicase, a viral coat protein and a viral transmissibility factor compatible with the chimeric nucleotide sequence. It is further preferred that the production cell has been stably transformed to contain coding sequences for a relicase, a viral coat protein and a viral transmissibility factor compatible with the chimeric nucleotide sequence.

Another preferred embodiment relates to a host having a chimeric nucleotide sequence which comprises a first nucleotide sequence which has substantial sequence homology to a viral nucleotide sequence and which is capable of replication and is biologically contained, and a second nucleotide sequence which is adjacent a viral promoter and which is a coding sequence capable of transcription in the hosst. The viral nucleotide sequence may be a prokaryotic viral nucleotide sequence or may be a eukaryotic viral nucleotide sequence. It is preferred that the viral nucleotide sequence is a plant viral nucleotide sequence. In the host the chimeric nucleotide sequence may be selected from the group consisting of DNA, RNA and cDNA. It is preferred that the viral nucleotide sequence lacks a biologically functional viral coat protein coding sequence, and optionally further lacks a biologically functional viral transmissibility factor coding sequence. In the host the viral promoter may be a viral coat protein promoter. It is preferred that the host has been stably transformed to contain a coding sequence for a replicase useful for the replication of the chimeric nucleotide sequence. It is further preferred that the host has been stably transformed to contain a coding sequence for a replicase useful for the replication of said chimeric nucleotide sequence.

Another preferred embodiment relates to a process for producing a product in a host, which comprises (a) infecting the host with a virus comprising a chimeric nucleotide sequence which comprises a first nucleotide sequence which has substantial sequence homology to a viral nucleotide sequence and which is capable of replication and is biologically contained, and a second nucleotide sequence which is adjacent a viral promoter and which is a coding sequence for said product capable of transcription in a host and (b) growing the infected host, whereby the host produces the product. Another preferred embodiment relates to a process of producing a product in a transgenic production organism, or part thereof, containing one or more coding sequences for (i) a viral replicase, (ii) a viral protein, (iii) a viral transmissibility factor or (iv) combinations thereof, said process comprising (a) infecting one or cells of the organism with an infectious virus, said virus comprising a chimeric nucleotide sequence which comprises a first nucleotide sequence which has substantial sequence homology to a viral nucleotide sequence and which is capable of replication and is biologically contained, and a second nucleotide sequence which is adjacent a viral promoter and which is a coding sequence for said product capable of transcription in a host and (b) growing the transgenic organism, whereby the transgenic organism produces the product of transcription of the coding sequence in the second nucleotide sequence. In the aforementioned processes the viral nucleotide sequences may be a prokaryotic viral nucleotide sequence or a eukaryotic viral nucleotide sequence. It is preferred that the viral nucleotide sequence is a plant viral nucleotide sequence. It is preferred that the chimeric nucleotide sequence is selected from the group consisting of DNA, RNA and cDNA. It is further preferred that the viral nucleotide sequence lacks a biologically functional viral coat protein coding sequence, and optionally further lacks a biologically functional viral transmissibility fator coding sequence. It is especially preferred that the viral nucleotide sequence further lacks a biologically functional viral transmissibility factor coding sequence. For the purposes of the afore-mentioned processes the viral promoter may be a viral coat protein promoter.

Another preferred embodiment relates to a product produced by the afore-mentioned processes, wherein the product is seletected from the group consisting of biopolymers , hormones and enzymes. Examples for the biopolymers are melanin or cyclodextrin. Examples for the enzyme are tyrosinase, cyclodextrin gluconotransferase or human tissue plasminogen activator.

In the above-mentioned processes it is preferred if the viral promoter is a viral coat protein promoter. It is further preferred that the host has been stably transformed to contain a coding sequence for a replicase useful for the replication of the chimeric nucleotide sequence.

Another preferred embodiment relates to a transgenic organism or part thereof, comprising cells which are host to a chimeric nucleotide sequence of an infectious virus and which contain a coding sequence for a viral replicase compatible with the chimeric nucleotide sequence, wherein the virus comprising a chimeric nucleotide sequence which comprises a first nucleotide sequence which has substantial sequence homology to a viral nucleotide sequence and which is capable of replication and is biologically contained, and a second nucleotide sequence which is adjacent a viral promoter and which is a coding sequence capable of transcription in a transgenic organism, said transgenic organism is capable of expressing one or more traits coded for by the chimeric nucleotide sequence. The viral nucleotide sequence may be a prokaryotic viral nucleotide sequence or a eukaryotic viral nucleotide sequence. It is preferred that the viral nucleotide sequence is a plant viral nucleotide sequence. In the transgenic organism the chimeric nucleotide sequence may be selected from the group consisting of DNA, RNA and cDNA. It is preferred that the viral nucleotide sequence further lacks a biologically functional viral transmissibility factor coding sequence. In the transgenic organism it is preferred that the viral promoter is a viral coat protein promoter.

## Claims

1. An animal production cell comprising:
(1) a first vector which comprises a chimeric nucleotide sequence comprising:
(a) a first nucleotide sequence having substantial sequence homology to an animal viral nucleotide sequence which is capable of replication and is biologically contained, wherein said viral nucleotide sequence lacks a biologically functional viral coat protein coding sequence;
(b) a second nucleotide sequence which is adjacent to a viral promoter and which is a coding sequence of interest capable of transcription in the animal cell;
and
(2) a second vector comprising a coding sequence for a biologically functional viral coat protein being compatible with the first nucleotide sequence.

2. The production cell of claim 1, wherein the nucleotide sequence of interest codes for the expression of one or more phenotypic traits.

3. The production cell of either of claims 1 or 2, wherein the first and/or second nucleotide sequence of the first vector is DNA, RNA or cDNA.

4. The production cell of any of claims 1 to 3, wherein the viral nucleotide sequence further lacks a coding sequence for a biologically functional viral transmissibility factor.

5. The production cell of any of claims 1 to 4, wherein the viral promoter is a viral coat protein promoter.

6. A process comprising:
(a) infecting an animal host with the first and second vectors as defined in any of claims 1 to 5,
(b) growing the infected animal to produce the product of the second nucleotide sequence contained in the first vector, and
(c) isolating the product of the second nucleotide sequence.

7. A process comprising:
(a) infecting an animal with the first vector as defined in any of claims 1 to 5, wherein the animal has been transformed in advance such that the coding sequence for the biologically functional viral coat protein is stably incorporated into the genome of the animal;
(b) growing the infected animal to produce the product of the second nucleotide sequence contained in the first vector, and
(c) isolating the product of the second nucleotide sequence.

8. The process of claim 6 or 7, wherein said product is an anti-sense RNA, a ribozyme, a protein, an enzyme or a complex biomolecule.

9. The process of claim 8, wherein the biomolecule is melanin or cyclodextrin.

10. The process of claim 9, wherein the enzyme is tyrosinase, cyclodextrin gluconotransferase or human tissue plasminogen activator.

11. An animal which has been infected with the first and second vectors as defined in any of claims 1 to 5 comprising:
(1) a first vector which comprises a chimeric nucleotide sequence comprising:
(a) a first nucleotide sequence having substantial sequence homology to an animal viral nucleotide sequence which is capable of replication and is biologically contained, wherein said viral nucleotide sequence lacks a biologically functional viral coat protein coding sequence;
(b) a second nucleotide sequence which is adjacent to a viral promoter and which is a coding sequence of interest capable of transcription in the animal;
and
(2) a second vector comprising a biologically functional viral coat protein coding sequence being compatible with the first nucleotide sequence.

12. The animal of claim 11, wherein the nucleotide sequence of interest codes for the expression of one or more phenotypic traits.

13. The animal of either of claims 11 or 17, wherein the first and/or second nucleotide sequence of the first vector is DNA, RNA or cDNA.

14. The animal of any of claims 11 to 13, wherein the viral nucleotide sequence further lacks a coding sequence for a biologically functional viral transmissibility factor.

15. The animal of any of claims 11 to 14, wherein the viral promoter is a viral coat protein promoter.
